(19) Europäisches Patentamt
European Patent Office
Office européen des brevets

(11) Veröffentlichungsnummer: **0 111 695**
**A2**

# (12) EUROPÄISCHE PATENTANMELDUNG

(21) Anmeldenummer: **83110788.3**

(22) Anmeldetag: **28.10.83**

(51) Int. Cl.³: **C 07 D 237/08**
C 09 K 3/34, C 07 D 237/14
C 07 D 237/12

(30) Priorität: **19.11.82 CH 6748/82**
**14.01.83 CH 208/83**
**21.09.83 CH 5132/83**

(43) Veröffentlichungstag der Anmeldung:
**27.06.84 Patentblatt 84/26**

(84) Benannte Vertragsstaaten:
**CH DE GB LI**

(71) Anmelder: **F. HOFFMANN-LA ROCHE & CO. Aktiengesellschaft**

**CH-4002 Basel(CH)**

(72) Erfinder: **Petrzilka, Martin, Dr.**
**Violaweg 74**
**CH-4303 Kaiseraugst(CH)**

(72) Erfinder: **Trickes, Georg, Dr.**
**Jakob Burckhardt-Strasse 9**
**D-7889 Grenzach-Wyhlen(DE)**

(74) Vertreter: **Cottong, Norbert A. et al,**
**Grenzacherstrasse 124 Postfach 3255**
**CH-4002 Basel(CH)**

(54) **Pyridazinderivate.**

(57) Verbindungen der Formel

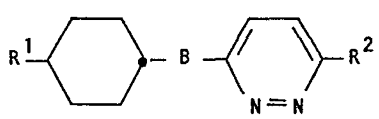

I

worin B die Gruppe $-C{\equiv}C-$ oder $-CH_2CH_2-$ darstellt, $R^1$ geradkettiges $C_1$-$C_{12}$-Alkyl bezeichnet und $R^2$ geradkettiges $C_1$-$C_{12}$Alkyl oder geradkettiges $C_1$-$C_{12}$-Alkoxy bedeutet, deren Herstellung und Verwendung in flüssigkristallinen Gemischen werden beschrieben.

EP 0 111 695 A2

F.HOFFMANN-LA ROCHE & CO. Aktiengesellschaft, Basel/Schweiz

RAN 6220/035K

1

# Pyridazinderivate

Die vorliegende Erfindung betrifft neue Pyridazinderivate der allgemeinen Formel

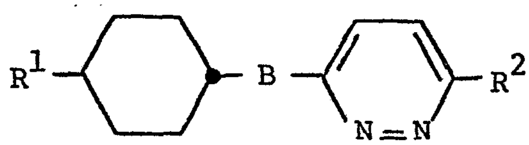

I

worin B die Gruppe -C≡C- oder $-CH_2CH_2-$ darstellt, $R^1$ geradkettiges $C_1$-$C_{12}$-Alkyl bezeichnet und $R^2$ geradkettiges $C_1$-$C_{12}$-Alkyl oder geradkettiges $C_1$-$C_{12}$-Alkoxy bedeutet.

Die Erfindung betrifft ebenfalls die Herstellung der Verbindungen der obigen Formel I, flüssigkristalline Gemische, die solche Verbindungen enthalten, sowie die Verwendung in elektro-optischen Vorrichtungen.

Im Rahmen der vorliegenden Erfindung bedeutet der Ausdruck "geradkettiges $C_1$-$C_{12}$-Alkyl" die Gruppen Methyl, Aethyl, Propyl, Butyl, Pentyl, Hexyl, Heptyl, Octyl, Nonyl, Decyl, Undecyl und Dodecyl und der Ausdruck "geradkettiges $C_1$-$C_{12}$-Alkoxy" die Gruppen Methoxy, Aethoxy, Propyloxy,

Zim/20.9.83

Butyloxy, Pentyloxy, Hexyloxy, Heptyloxy, Octyloxy, Nonyloxy, Decyloxy, Undecyloxy und Dodecyloxy.

Die dielektrische Anisotropie $\Delta\varepsilon$ einer Verbindung oder Mischung ist die Differenz zwischen der Dielektrizitätskonstante entlang der Moleküllängsachse ($\varepsilon_{\parallel}$) und der Dielektrizitätskonstante senkrecht dazu ($\varepsilon_{\perp}$). Im Falle nicht flüssigkristalliner Komponenten bedeutet der Ausdruck "dielektrische Anisotropie" im Rahmen der vorliegenden Erfindung den extrapolierten Wert (aus flüssigkristallinen Gemischen, welche diese Komponente enthalten) der dielektrischen Anisotropie bei einer Temperatur, die 10°C unter dem extrapolierten (virtuellen) Klärpunkt liegt.

Nematische und cholesterische Flüssigkristalle mit negativer Anisotropie der Dielektrizitätskonstante ($\Delta\varepsilon = \varepsilon_{\parallel} - \varepsilon_{\perp} < 0$) werden in einem elektrischen Feld mit ihren Moleküllängsachsen senkrecht zur Feldrichtung ausgerichtet. Dieser Effekt ist bekannt und wird zur Steuerung der optischen Transparenz in verschiedenen Flüssigkristallanzeigen ausgenützt, so z.B. in Flüssigkristallzellen vom Lichtstreuungstyp (dynamische Streuung), vom sogenannten DAP-Typ (Deformation aufgerichteter Phasen) oder vom Gast/Wirt-Typ (guest host interaction).

Bei der "Guest/Host-Zelle" handelt es sich im wesentlichen um einen Kondensator, wobei mindestens eine Elektrode lichtdurchlässig ist und das Dielektrikum von einem nematischen oder cholesterischen Flüssigkristall gebildet wird, welcher einen oder mehrere dichroitische Farbstoffe enthält. Da die verwendbaren Farbstoffe meist positiven Dichroismus aufweisen, d.h. das Uebergangsmoment der Absorption von sichtbarem Licht annähernd in Richtung der Moleküllängsachse liegt, entspricht die Ausrichtung des Flüssigkristalls mit den Moleküllängsachsen parallel zur Plattenoberfläche im allgemeinen dem farbigen Zustand und die homöotrope Ausrichtung (Moleküllängsachsen senkrecht zur Plattenoberfläche) dem farblosen Zustand der

Zelle. Bei Verwendung eines Flüssigkristalls mit positiver dielektrischer Anisotropie wird dessen homogene Orientierung (welche durch Behandlung der Plattenoberfläche erreicht wird) durch Anlegen einer Spannung homöotrop ausgerichtet, d.h. die Zelle wird von "farbig" auf "farblos" geschaltet. Auf diese Weise können farblose Zeichen auf farbigem Untergrund angezeigt werden. Demgegenüber wird bei Verwendung eines Flüssigkristalls mit negativer dielektrischer Anisotropie dessen homöotrope Orientierung (welche durch Behandlung der Plattenoberfläche erreicht wird) durch Anlegen einer Spannung parallel zu den Elektrodenoberflächen ausgerichtet, wodurch die Anzeige farbiger Bildelemente auf farblosem Untergrund ermöglicht wird.

Ferner wurde zur Verbesserung des Multiplexverhältnisses bei der Multiplex-Ansteuerung von Flüssigkristallanzeigen, insbesondere von Drehzellen und Guest/Host-Zellen, eine Zwei-Frequenz-Matrix-Adressierung vorgeschlagen (z.B. Deutsche Offenlegungsschriften 2 856 134 und 2 907 940). Hierbei wird der Umstand ausgenützt, dass die dielektrische Anisotropie von Flüssigkristallen, welche beim Anlegen einer niederfrequenten Spannung eine positive Anisotropie der Dielektrizitätskonstante besitzen, bei hohen Frequenzen negativ wird. Um die kapazitiven Verluste klein zu halten, sollte die "Cross-over-Frequenz" $f_c$ (dielektrische Relaxationsfrequenz, bei welcher $\varepsilon_{\parallel} = \varepsilon_{\perp}$ wird) derartiger Flüssigkristalle möglichst niedrig sein und nicht über etwa 20 kHz liegen. Ferner sollte der absolute Betrag der dielektrischen Anisotropie sowohl unterhalb als auch oberhalb der Cross-over-Frequenz möglichst gross sein. Es hat sich jedoch gezeigt, dass die Substanzen, welche für die Zwei-Frequenz-Adressierung besonders geeignet sind, bei Frequenzen oberhalb der Cross-over-Frequenz im allgemeinen eine kleinere absolute dielektrische Anisotropie aufweisen als unterhalb der Cross-over-Frequenz. Dieser Nachteil könnte behoben werden durch Zusatz von Verbindungen mit negativer dielektrischer Anisotropie und geeignetem Relaxationsverhalten.

Es sind bereits eine Reihe von flüssigkristallinen Verbindungen mit schwach negativer dielektrischer Anisotropie bekannt. Hingegen sind noch relativ wenig Flüssigkristallkomponenten mit grosser negativer Anisotropie der Dielektrizitätskonstante bekannt. Zudem weisen letztere im allgemeinen Nachteile auf, wie z.B. schlechte Löslichkeit in Mischungen, hohe Viskosität, hohe Schmelzpunkte, starke smektische Tendenzen, chemische Instabilität oder starke Klärpunktsdepressionen in Mischungen. Es besteht daher ein Bedarf an weiteren Verbindungen mit negativer dielektrischer Anisotropie, die es erlauben, die Eigenschaften von Mischungen für verschiedenste elektro-optische Anwendungen weiter zu verbessern.

Es wurde nun gefunden, dass die erfindungsgemässen Verbindungen eine dielektrische Anisotropie von etwa -9, eine gute Löslichkeit in bekannten Flüssigkristallmischungen und eine niedrige Viskosität aufweisen. Sie sind farblos und besitzen eine gute chemische und photochemische Stabilität. Ferner können mit den erfindungsgemässen Verbindungen Mischungen hergestellt werden, welche ein verbessertes Schmelzverhalten und keine oder nur geringe smektische Tendenzen aufweisen. Obwohl die erfindungsgemässen Verbindungen meist nicht selbst flüssigkristallin sind, ergeben sie in Mischungen im allgemeinen keine oder nur geringe Klärpunktsdepressionen. Sie sind somit besonders geeignet als Komponenten von Flüssigkristallmischungen mit negativer dielektrischer Anisotropie und als Komponenten von Flüssigkristallmischungen, welche bei der Zwei-Frequenz-Matrix-Adressierung verwendet werden. Sie können aber auch in Mischungen mit positiver dielektrischer Anisotropie verwendet werden, beispielsweise um die Schwellenspannung an die verwendete elektro-optische Zelle anzupassen.

Je nach Bedeutung der Gruppe B umfasst die obige Formel I Verbindungen der folgenden allgemeinen Formeln

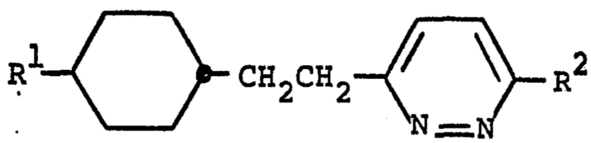

Ia

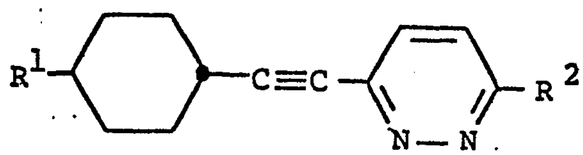

Ib

Vorzugsweise bedeutet $R^2$ in den obigen Formeln I, Ia und Ib geradkettiges $C_1$-$C_{12}$-Alkyl. Bevorzugte Reste $R^1$ sind die geradkettigen $C_3$-$C_7$-Alkylreste. Bevorzugte Alkyl- und Alkoxyreste $R^2$ sind geradkettiges $C_1$-$C_7$-Alkyl (insbesondere geradkettiges $C_2$-$C_7$-Alkyl) und geradkettiges $C_1$-$C_6$-Alkoxy.

Beispiele bevorzugter erfindungsgemässer Verbindungen sind die Verbindungen der Formel I, worin $R^1$, B und $R^2$ die in Tabelle 1 gegebenen Bedeutungen haben, sowie die weiteren in den Synthesebeispielen genannten Verbindungen der Formel I.

Tabelle 1

| $R^1$ | B | $R^2$ |
|---|---|---|
| $C_5H_{11}$ | $-CH_2CH_2-$ | $C_2H_5$ |
| $C_7H_{15}$ | $-CH_2CH_2-$ | $C_2H_5$ |
| $C_4H_9$ | $-CH_2CH_2-$ | $C_3H_7$ |
| $C_5H_{11}$ | $-CH_2CH_2-$ | $C_3H_7$ |
| $C_7H_{15}$ | $-CH_2CH_2-$ | $C_3H_7$ |
| $C_3H_7$ | $-CH_2CH_2-$ | $C_5H_{11}$ |
| $C_4H_9$ | $-CH_2CH_2-$ | $C_5H_{11}$ |
| $C_5H_{11}$ | $-CH_2CH_2-$ | $C_5H_{11}$ |

| | | |
|---|---|---|
| $C_3H_7$ | $-CH_2CH_2-$ | $C_7H_{15}$ |
| $C_5H_{11}$ | $-CH_2CH_2-$ | $OC_2H_5$ |
| $C_7H_{15}$ | $-CH_2CH_2-$ | $OC_2H_5$ |
| $C_3H_7$ | $-CH_2CH_2-$ | $OC_4H_9$ |
| $C_4H_9$ | $-CH_2CH_2-$ | $OC_4H_9$ |
| $C_5H_{11}$ | $-CH_2CH_2-$ | $OC_4H_9$ |
| $C_3H_7$ | $-CH_2CH_2-$ | $OC_6H_{13}$ |
| $C_3H_7$ | $-C{\equiv}C-$ | $C_3H_7$ |
| $C_3H_7$ | $-C{\equiv}C-$ | $C_4H_9$ |
| $C_3H_7$ | $-C{\equiv}C-$ | $C_5H_{11}$ |
| $C_3H_7$ | $-C{\equiv}C-$ | $C_6H_{13}$ |
| $C_3H_7$ | $-C{\equiv}C-$ | $C_7H_{15}$ |
| $C_3H_7$ | $-C{\equiv}C-$ | $OCH_3$ |
| $C_3H_7$ | $-C{\equiv}C-$ | $OC_2H_5$ |
| $C_3H_7$ | $-C{\equiv}C-$ | $OC_3H_7$ |
| $C_3H_7$ | $-C{\equiv}C-$ | $OC_4H_9$ |
| $C_3H_7$ | $-C{\equiv}C-$ | $OC_5H_{11}$ |
| $C_3H_7$ | $-C{\equiv}C-$ | $OC_6H_{13}$ |
| $C_4H_9$ | $-C{\equiv}C-$ | $C_3H_7$ |
| $C_4H_9$ | $-C{\equiv}C-$ | $C_5H_{11}$ |
| $C_4H_9$ | $-C{\equiv}C-$ | $C_7H_{15}$ |
| $C_4H_9$ | $-C{\equiv}C-$ | $OCH_3$ |
| $C_4H_9$ | $-C{\equiv}C-$ | $OC_2H_5$ |
| $C_4H_9$ | $-C{\equiv}C-$ | $OC_3H_7$ |
| $C_4H_9$ | $-C{\equiv}C-$ | $OC_4H_9$ |
| $C_5H_{11}$ | $-C{\equiv}C-$ | $CH_3$ |
| $C_5H_{11}$ | $-C{\equiv}C-$ | $C_2H_5$ |
| $C_5H_{11}$ | $-C{\equiv}C-$ | $C_3H_7$ |
| $C_5H_{11}$ | $-C{\equiv}C-$ | $C_4H_9$ |
| $C_5H_{11}$ | $-C{\equiv}C-$ | $C_5H_{11}$ |
| $C_5H_{11}$ | $-C{\equiv}C-$ | $C_6H_{13}$ |
| $C_5H_{11}$ | $-C{\equiv}C-$ | $C_7H_{15}$ |
| $C_5H_{11}$ | $-C{\equiv}C-$ | $OCH_3$ |
| $C_5H_{11}$ | $-C{\equiv}C-$ | $OC_2H_5$ |
| $C_5H_{11}$ | $-C{\equiv}C-$ | $OC_3H_7$ |
| $C_5H_{11}$ | $-C{\equiv}C-$ | $OC_4H_9$ |
| $C_5H_{11}$ | $-C{\equiv}C-$ | $OC_5H_{11}$ |
| $C_5H_{11}$ | $-C{\equiv}C-$ | $OC_6H_{13}$ |

| | | |
|---|---|---|
| $C_6H_{13}$ | $-C\equiv C-$ | $C_3H_7$ |
| $C_6H_{13}$ | $-C\equiv C-$ | $C_5H_{11}$ |
| $C_6H_{13}$ | $-C\equiv C-$ | $OCH_3$ |
| $C_6H_{13}$ | $-C\equiv C-$ | $OC_2H_5$ |
| $C_6H_{13}$ | $-C\equiv C-$ | $OC_4H_9$ |
| $C_7H_{15}$ | $-C\equiv C-$ | $CH_3$ |
| $C_7H_{15}$ | $-C\equiv C-$ | $C_2H_5$ |
| $C_7H_{15}$ | $-C\equiv C-$ | $C_3H_7$ |
| $C_7H_{15}$ | $-C\equiv C-$ | $C_4H_9$ |
| $C_7H_{15}$ | $-C\equiv C-$ | $C_5H_{11}$ |
| $C_7H_{15}$ | $-C\equiv C-$ | $OCH_3$ |
| $C_7H_{15}$ | $-C\equiv C-$ | $OC_2H_5$ |
| $C_7H_{15}$ | $-C\equiv C-$ | $OC_3H_7$ |
| $C_7H_{15}$ | $-C\equiv C-$ | $OC_4H_9$ |

Die Verbindungen der Formel I können erfindungsgemäss dadurch hergestellt werden, dass man

a) zur Herstellung der Verbindungen der Formel I, worin B die Gruppe $-C\equiv C-$ darstellt, eine Verbindung der allgemeinen Formel

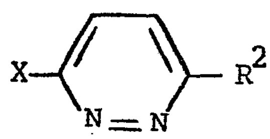

IV

worin X Chlor, Brom oder Jod bezeichnet und $R^2$ die obige Bedeutung hat,

in Gegenwart von Kupfer-(I)-jodid, einer Triphenylphosphinpalladiumverbindung und eines Amins mit einer Verbindung der allgemeinen Formel

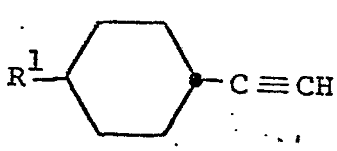

V

worin $R^1$ die oben gegebene Bedeutung hat,

umsetzt, oder

b) zur Herstellung der Verbindungen der Formel I, worin B die Gruppe -C≡C- und $R^2$ geradkettiges $C_1$-$C_{12}$-Alkoxy bedeutet, eine Verbindung der allgemeinen Formel

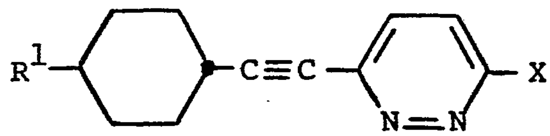

VI

worin X Chlor, Brom oder Jod bedeutet und $R^1$ die obige Bedeutung hat,

mit Alkalimetallalkanolat umsetzt, oder

c) zur Herstellung der Verbindungen der Formel I, worin B die Gruppe $-CH_2CH_2-$ und $R^2$ geradkettiges $C_1$-$C_{12}$-Alkyl bedeuten, eine Verbindung der allgemeinen Formel

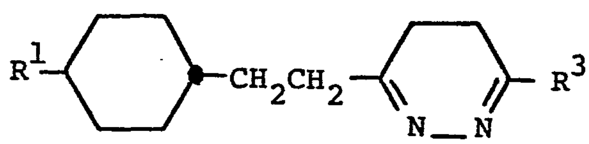

II

worin $R^3$ geradkettiges $C_1$-$C_{12}$-Alkyl bezeichnet und $R^1$ die obige Bedeutung hat,

oder ein tautomeres Dihydropyridazin oxidiert, oder

d) zur Herstellung der Verbindungen der Formel I, worin B die Gruppe $-CH_2CH_2-$ und $R^2$ geradkettiges $C_1$-$C_{12}$-Alkoxy bedeuten, eine Verbindung der allgemeinen Formel

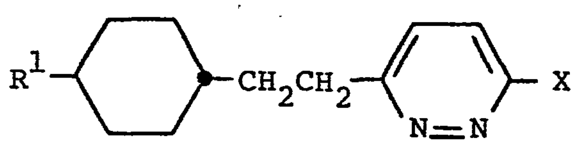

III

worin X Chlor, Brom oder Jod bezeichnet und $R^1$ die obige Bedeutung hat,

mit Alkalimetallalkanolat umsetzt, oder

e) zur Herstellung der Verbindungen der Formel I, worin B die Gruppe $-CH_2CH_2-$ darstellt, eine Verbindung der Formel I, worin B die Gruppe -C≡C- darstellt, katalytisch hydriert.

Die Umsetzung einer Verbindung der Formel IV mit einer Verbindung der Formel V in Gegenwart von Kupfer-(I)-jodid, einer Triphenylphosphin-palladiumverbindung und eines Amins (Verfahrensvariante a) kann in an sich bekannter Weise durchgeführt werden, beispielsweise in analoger Weise zu den in Chem. Pharm. Bull. 28, 3488 (1980), Synthesis 627 (1980) und J. Org. Chem. 46, 2280 (1981) beschriebenen Reaktionen. Geeignete Triphenylphosphin-palladiumverbindungen sind Bis-(triphenylphosphin)-palladium-(II)-dichlorid, Tetrakis-(triphenylphosphin)-palladium und dergleichen. Als Basen eignen sich beispielsweise Triäthylamin, Piperidin, Diäthylamin und dergleichen. Tertiäre Amine sind bevorzugt. Temperatur und Druck sind keine kritischen Aspekte in dieser Reaktion. Zweckmässigerweise werden Atmosphärendruck und eine Temperatur zwischen 0°C und etwa 40°C angewendet. Vorzugsweise bedeutet X in Formel IV Chlor oder Brom, insbesondere Brom.

Die Umsetzung einer Verbindung der Formel VI oder III mit Alkalimetallalkoholat, nämlich dem entsprechenden Alkalimetallalkanolat, (Verfahrensvarianten b und d) kann in an sich bekannter Weise erfolgen. Bevorzugtes Alkalimetall ist Natrium. Die Reaktion kann in einem inerten organischen Lösungsmittel, beispielsweise einem Aether oder einem gesättigten oder aromatischen Kohlenwasserstoff, wie Diäthyläther, Dioxan, Benzol, Toluol, Hexan und dergleichen, erfolgen. Vorzugsweise wird jedoch der dem Alkoholat entsprechende Alkohol als Lösungsmittel verwendet (gegebenenfalls in Kombination mit einem inerten organischen Lösungsmittel). Die alkoholische Lösung des Alkoholates wird hierbei zweckmässig durch Umsetzung eines Ueberschusses des Alkohols mit Natrium, Natriumhydrid, Kaliumhydrid und dergleichen hergestellt. Temperatur und Druck sind nicht kritisch. Zweckmässigerweise werden jedoch Atmosphärendruck und eine Temperatur zwischen Raumtemperatur und der Rückflusstemperatur des Reaktionsgemisches, vorzugsweise etwa 40°C bis 60°C angewendet. Vorzugsweise bedeutet X in Formel III und VI Chlor oder Brom.

Die Oxidation einer Verbindung der Formel II (Verfahrensvariante c) kann in an sich bekannter Weise z.B. mit 2,3-Dichloro-5,6-dicyanobenzochinon in Dioxan, mit Natriumnitrit in Eisessig und Aethanol, mit Isopentylnitrit in Eisessig und dergleichen durchgeführt werden. Temperatur und Druck sind keine kritischen Aspekte in dieser Reaktion. Zweckmässigerweise werden jedoch Atmosphärendruck und eine Temperatur zwischen Raumtemperatur und Rückflusstemperatur angewendet. Vorzugsweise werden die Verbindungen der Formel II jedoch durch katalytische Dehydrierung in an sich bekannter Weise zu Verbindungen der Formel I oxidiert. Die Dehydrierung kann mit irgendeinem in Dehydrierungsreaktionen üblicherweise verwendeten Katalysator, wie Palladium, Platin und dergleichen (gegebenenfalls auf einem inerten Trägermaterial, z.B. Kohle) durchgeführt werden. Bevorzugter Katalysator ist Palladium. Als Lösungsmittel können irgendwelche inerte organische Lösungsmittel, wie Alkohole, Aether, Ester, Carbonsäure und dergleichen, beispielsweise Aethanol, Dioxan, Essigsäureäthylester oder Eisessig verwendet werden. Bevorzugtes Lösungsmittel ist Aethanol. Temperatur und Druck sind keine kritischen Aspekte in dieser Reaktion. Zweckmässigerweise werden eine Temperatur zwischen Raumtemperatur und der Rückflusstemperatur des Reaktionsgemisches und Atmosphärendruck angewendet.

Die Verbindungen der Formel II können durch Wanderung der Doppelbindungen im Dihydropyridazin-Ring zu tautomeren Verbindungen umlagern. Derartige Umlagerungen können z.B. durch Anwesenheit einer Spur Säure oder Base ausgelöst werden. Da die tautomeren Dihydropyridazine jedoch ebenfalls unter den obigen Bedingungen zu Ver-

bindungen der Formel I oxidiert werden können, kann gemäss Verfahrensvariante c) sowohl eine Verbindung der Formel II als auch ein tautomeres Dihydropyridazin oder ein Gemisch solcher Verbindungen umgesetzt werden.

Die katalytische Hydrierung einer Verbindung der Formel I, worin B die Gruppe -C≡C- darstellt, d.h. einer Verbindung der Formel Ib, (Verfahrensvariante e) kann in an sich bekannter Weise in Gegenwart von üblichen Hydrierkatalysatoren durchgeführt werden. Bevorzugte Katalysatoren sind Palladium, Platin und dergleichen, gegebenenfalls auf einem inerten Trägermaterial (z.B. Kohle). Die Hydrierung wird zweckmässig in einem inerten organischen Lösungsmittel durchgeführt, beispielsweise in einem gesättigten Alkohol, Aether, Ester, Kohlenwasserstoff oder einer gesättigten Carbonsäure, wie z.B. Aethanol, Dioxan, Essigsäureäthylester, Hexan, Eisessig und dergleichen. Temperatur und Druck sind keine kritischen Aspekte in dieser Reaktion. Zweckmässigerweise werden eine Temperatur zwischen Raumtemperatur und der Siedetemperatur des Reaktionsgemisches und ein Druck von etwa 1 bis 5 Atmosphären angewendet.

Die Ausgangsmaterialien derFormeln II-VI sind neu und bilden ebenfalls Gegenstand der vorliegenden Erfindung.

Die Verbindungen der Formeln II, III und V und die Verbindungen der Formel IV, worin $R^2$ geradkettiges $C_1$-$C_{12}$-Alkyl bedeutet, können in an sich bekannter Weise hergestellt werden, beispielsweise anhand der folgenden Reaktionsschemata A-D, worin $R^1$ und $R^3$ geradkettiges $C_1$-$C_{12}$-Alkyl bedeuten und X Chlor, Brom oder Jod, vorzugsweise Chlor oder Brom bezeichnet.

## Schema A

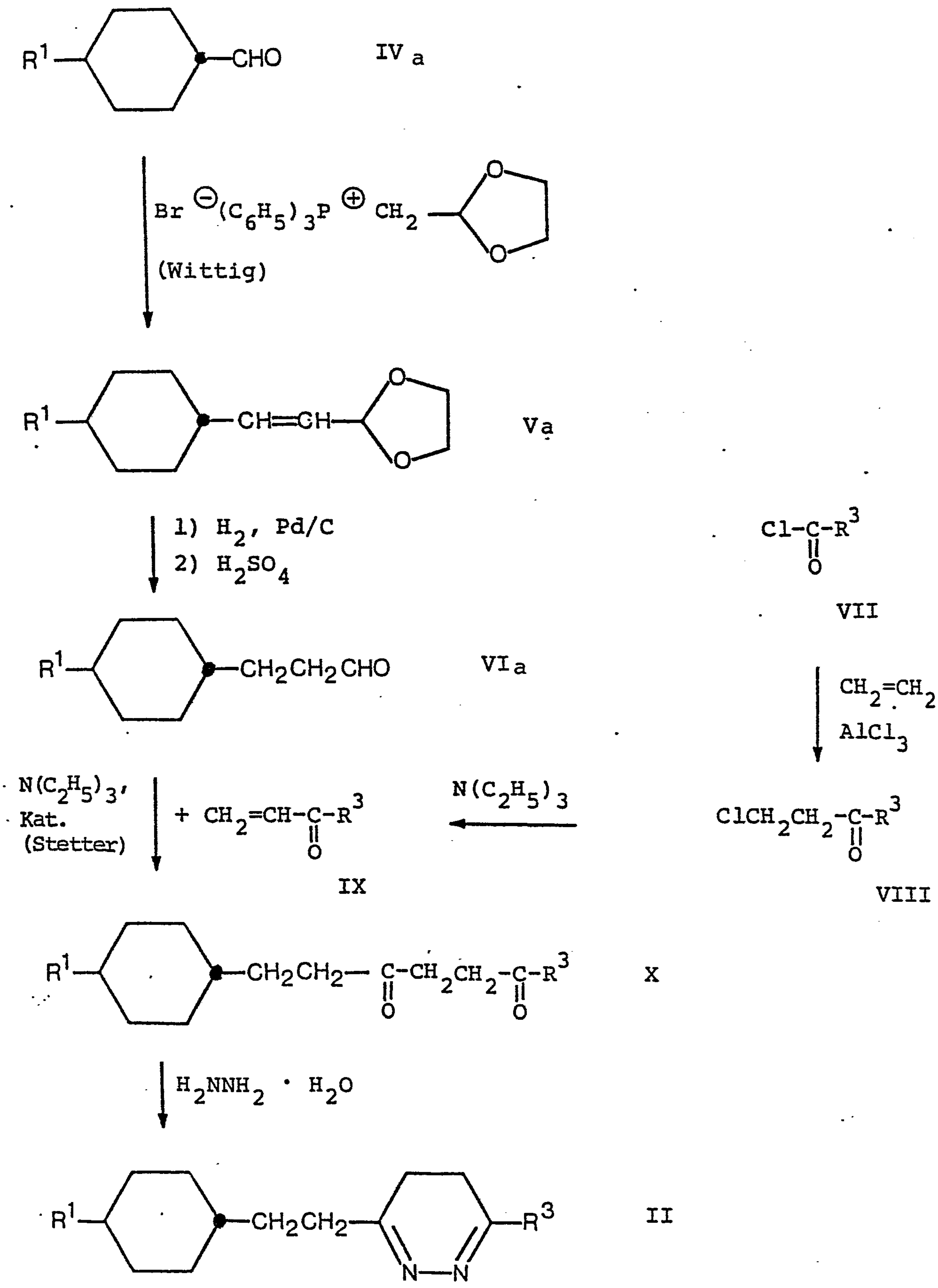
IV a
Br ⊖ $(C_6H_5)_3P$ ⊕ $CH_2$
(Wittig)
Va
1) $H_2$, Pd/C
2) $H_2SO_4$
VII
VIa
$CH_2=CH_2$
$AlCl_3$
$N(C_2H_5)_3$,
Kat.
(Stetter)
$N(C_2H_5)_3$
IX
VIII
X
$H_2NNH_2 \cdot H_2O$
II

Schema B

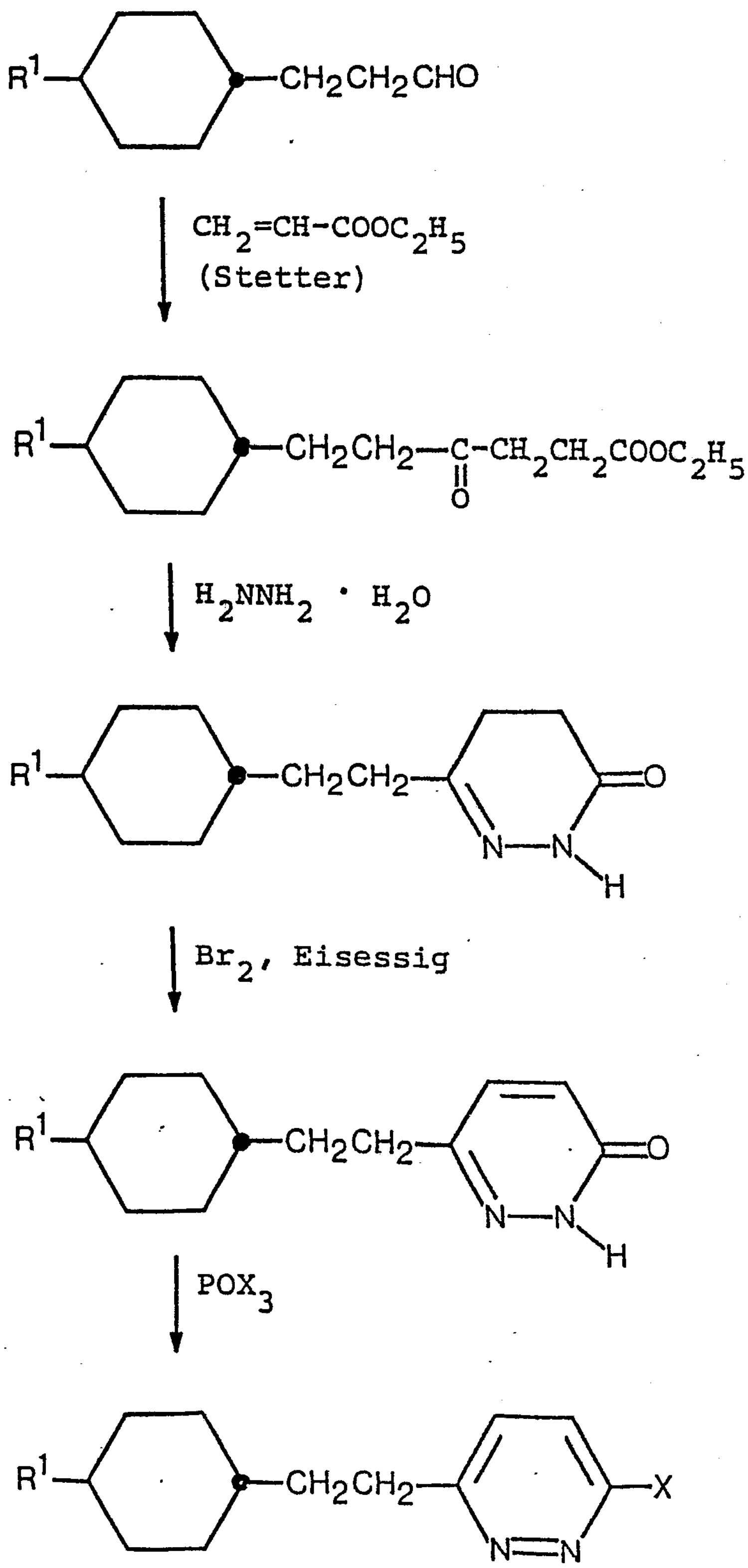


VIa

XI

XII

XIII

III

## Schema C

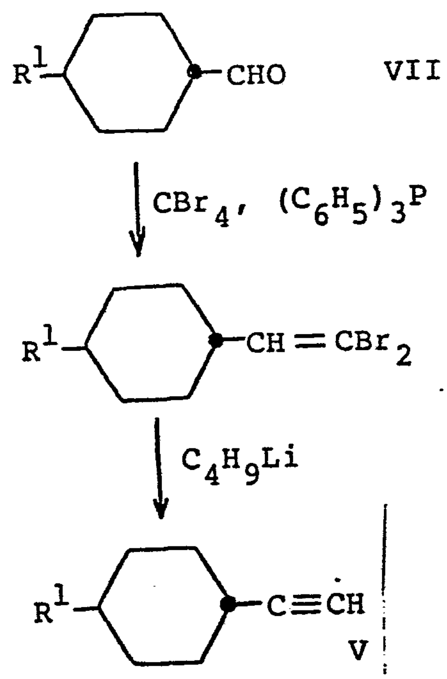


VII a

VIII a

V

Schema D

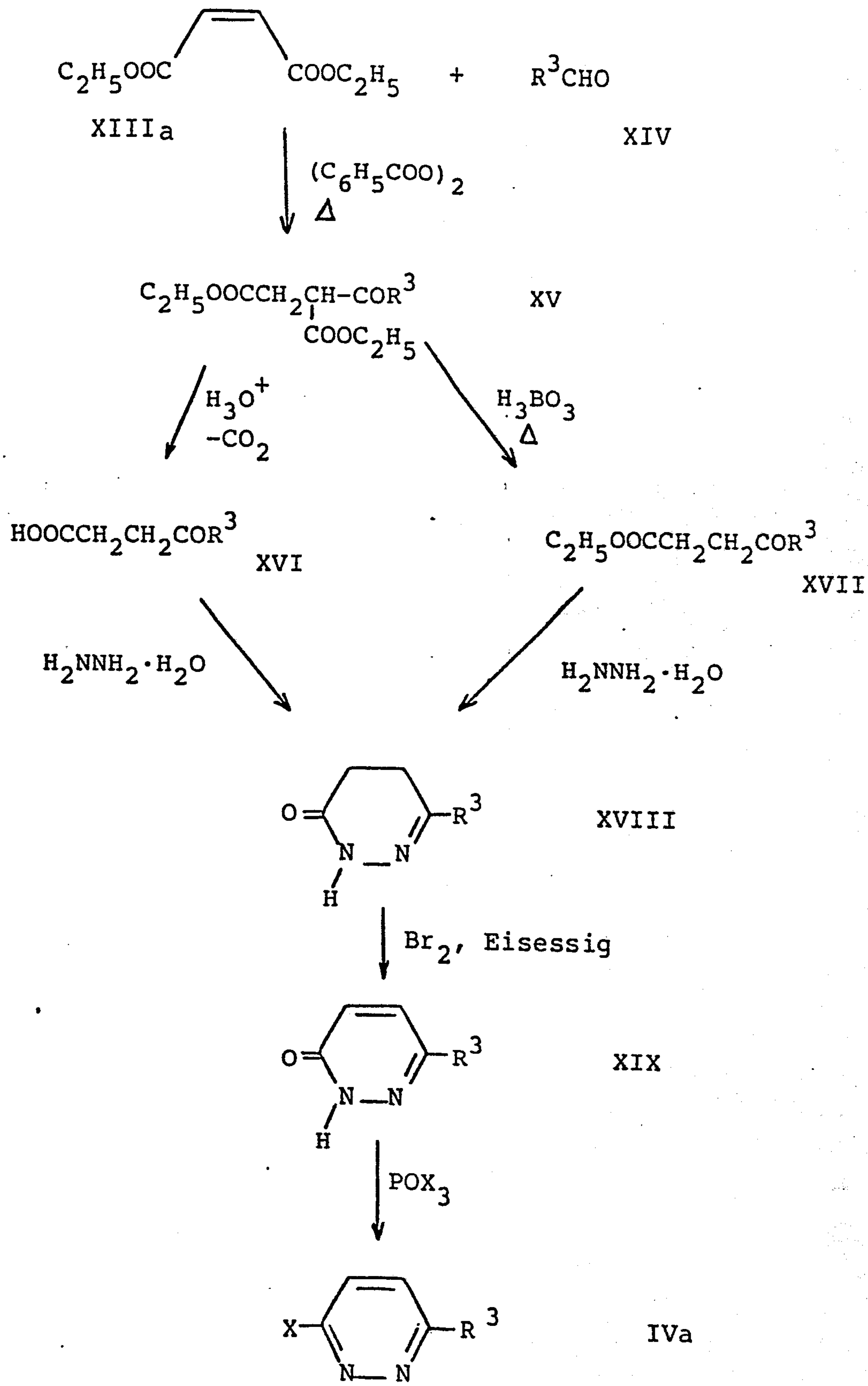
XIIIa
XIV
$(C_6H_5COO)_2$
Δ
XV
$H_3O^+$
$-CO_2$
$H_3BO_3$
Δ
XVI
XVII
$H_2NNH_2 \cdot H_2O$
$H_2NNH_2 \cdot H_2O$
XVIII
$Br_2$, Eisessig
XIX
$POX_3$
IVa

Die Ausgangssubstanzen der Formeln IVa und VII sind bekannte oder Analoge bekannter Verbindungen und können nach bekannten Methoden hergestellt werden.

Die Addition eines Aldehyds der Formel VIa an eine Doppelbindung gemäss Schema A bzw. B kann nach der Methode von Stetter (Chem. Ber. 114 (1981) 581) in Gegenwart eines 1,3-Thiazoliumsalz-Katalysators erfolgen. Bevorzugter Katalysator ist in Schema A 3-Benzyl-5-(2-hydroxyäthyl)-4-methyl-1,3-thiazoliumchlorid und in Schema B 3-(2-Aethoxyäthyl)- 5- (2-hydroxyäthyl)-4-methyl-1,3-thiazoliumbromid.

Die Verbindungen der Formel II können wie bereits weiter oben erwähnt auch in tautomerer Form bzw. als Gemisch tautomerer Formen vorliegen.

Die Verbindungen der Formel XVII können auch nach der von Stetter in Chem. Ber. 113, 690 (1980) beschriebenen Methode aus Acrylsäureäthylester und einem Aldehyd $R^3CHO$ erhalten werden.

Die Verbindungen der Formeln Ib und IV, worin $R^2$ Alkoxy bedeutet, und die Verbindungen der Formel VI können aus 3,6-Dihalogenopyridazinen, z.B. dem 3,6-Dichlorpyridazin, erhalten werden. Auf diesem Wege können ausgehend vom 3,6-Dihalogenopyridazin in 2 Stufen, nämlich durch Umsetzung mit einer Verbindung der Formel V in analoger Weise zu Verfahrensvariante a) und durch Verätherung mit einem Alkalimetallalkoholat in analoger Weise zu Verfahrensvariante b) Pyridazine der Formel I erhalten werden. Diese 2 Stufen können in beliebiger Reihenfolge durchgeführt werden, d.h. die Herstellung kann via Verbindungen der Formel IV oder VI verlaufen.

Die erfindungsgemässen Verbindungen können in Form ihrer Gemische mit einem flüssigkristallinen Trägermaterial verwendet werden. Das Trägermaterial kann übliche

Flüssigkristallkomponenten enthalten, wie z.B. Substanzen aus den Klassen der Schiffschen Basen, Azo- und Azoxybenzole, Phenylbenzoate, Cyclohexancarbonsäurephenylester und -cyclohexylester, Bi- und Terphenyle, Phenylcyclohexane, Cyclohexylbiphenyle, Phenyl- und Diphenylpyrimidine, Cyclohexyl-phenylpyrimidine, Phenyldioxane, Phenylbicyclo[2.2.2]octane, Phenylpyridazine und dergleichen. Derartige Substanzen sind dem Fachmann bekannt und viele davon sind zudem im Handel erhältlich. Bei der Herstellung der Mischungen sollte jedoch darauf geachtet werden, dass geeignete flüssigkristalline Verbindungen in ausreichender Menge verwendet werden, damit die Gesamtmischung einen genügend grossen Mesophasenbereich aufweist.

Die erfindungsgemässen Mischungen enthalten zweckmässigerweise etwa 1-50 Gew.-% und vorzugsweise etwa 3-30 Gew.-% an Verbindungen der Formel I.

Grundsätzlich können die erfindungsgemässen Verbindungen in beliebigen flüssigkristallinen Gemischen eingesetzt werden, so z.B. auch in Gemischen mit positiver dielektrischer Anisotropie zwecks Anpassung der dielektrischen Anisotropie an die verwendete Zelle. Vorzugsweise werden die erfindungsgemässen Verbindungen jedoch in Mischungen mit negativer dielektrischer Anisotropie oder in Mischungen, welche für die Zwei-Frequenz-Ansteuerung geeignet sind, verwendet. Derartige Mischungen können in an sich bekannter Weise hergestellt werden.

Die erfindungsgemässen Mischungen für die Zwei-Frequenz-Matrix-Adressierung sind vorzugsweise dadurch gekennzeichnet, dass sie aus 3 Komponenten A, B und C bestehen, welche jeweils eine oder mehrere Verbindungen enthalten, und dass Komponente A eine Viskosität von höchstens 40 cP, einen Klärpunkt von mindestens 40°C und eine dielektrische Anisotropie zwischen -2 und +1 aufweist, Komponente B eine dielektrische Anisotropie unterhalb -2 besitzt und mindestens eine Verbindung der Formel I

enthält, und Komponente C eine dielektrische Anisotropie oberhalb +10, einen Klärpunkt von mindestens 100°C und eine Cross-over-Frequenz im Gesamtgemisch von höchstens 15 kHz bei 20°C aufweist.

Derartige Mischungen bestehen bevorzugt aus mindestens etwa 30 Gew.-% Komponente A, etwa 3-50 Gew.-% Komponente B und etwa 5-40 Gew.-% Komponente C und besonders bevorzugt aus etwa 30-87 Gew.-% Komponente A, etwa 3-40 Gew.-% Komponente B und etwa 10-30 Gew.-% Komponente C.

Verbindungen und Mischungen mit den oben, für die Komponenten A, B und C geforderten Eigenschaften sind dem Fachmann grundsätzlich bekannt. Die Gesamtmischung muss nematische oder cholesterische Eigenschaften aufweisen. Komponente A kann nematisch oder cholesterisch und Komponente C nematisch, cholesterisch oder - solange die Gesamtmischung nicht smektisch wird - auch smektisch sein.

Verbindungen und Mischungen, die sich als Komponente A eignen, sind in grosser Zahl bekannt und vielfach auch im Handel erhältlich. Vorzugsweise enthält die Komponente A eine oder mehrere Verbindungen der folgenden allgemeinen Formeln:

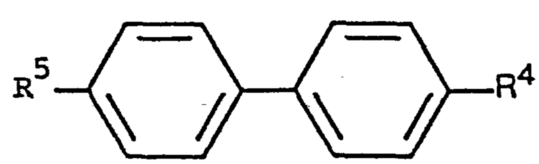

XXI

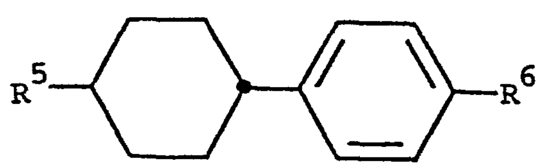

XXII

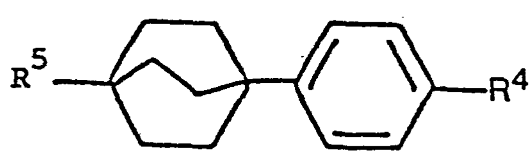

XXIII

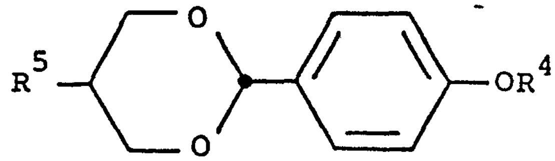

XXIV

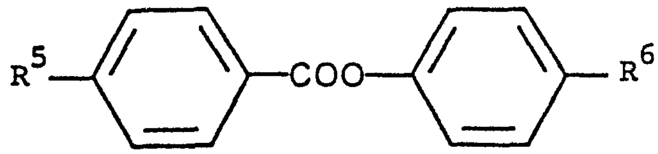

XXV

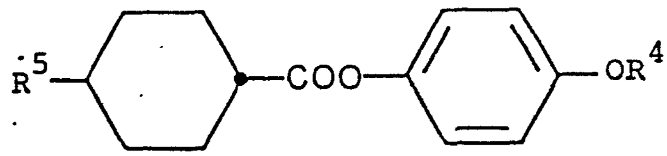

XXVI

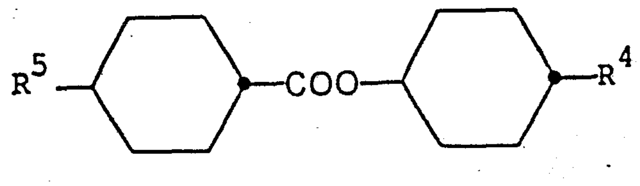

XXVII

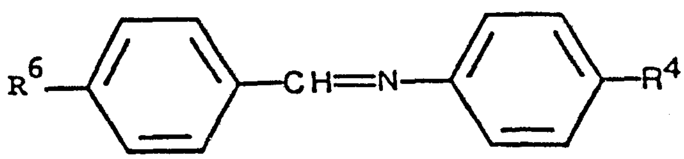

XXVIII

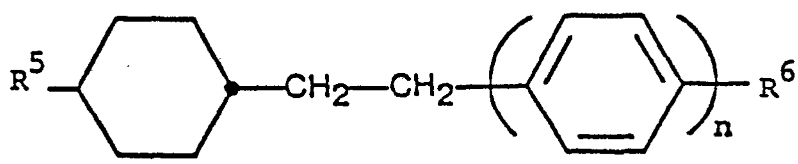

XXIX

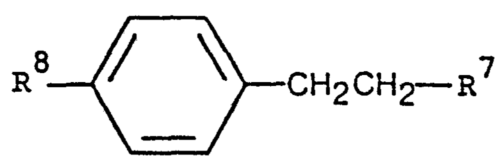

XXX

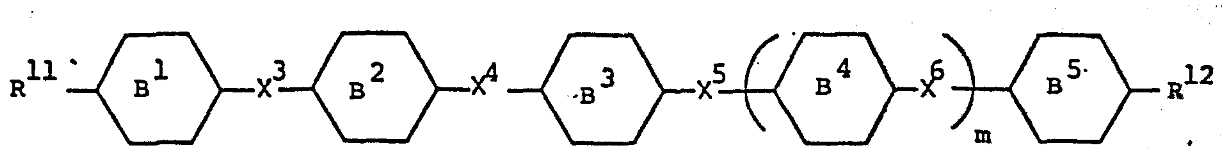

XXXI

worin $R^4$ und $R^5$ geradkettiges $C_1$-$C_8$-Alkyl, $R^6$ geradkettiges $C_1$-$C_8$-Alkyl oder $C_1$-$C_8$-Alkoxy und n 1 oder 2 bedeuten; $R^8$ trans-4-Alkylcyclohexyl, 4'-Alkyl-4-biphenylyl, p-(trans-4-Alkylcyclohexyl)phenyl,

2-(trans-4-Alkylcyclohexyl)äthyl oder p-[2-(trans-4-Alkylcyclohexyl)äthyl]phenyl und $R^7$ trans-4-Alkylcyclohexyl darstellen, oder $R^8$ trans-4-Alkylcyclohexyl und $R^7$ p-(trans-4-Alkylcyclohexyl)phenyl, p-[2-(trans-4-Alkylcyclohexyl)äthyl]phenyl oder 4'-(trans-4-Alkylcyclohexyl)-4-biphenylyl darstellen, oder $R^8$ p-Alkylphenyl und $R^7$ p-[2-(trans-4-Alkylcyclohexyl)äthyl]phenyl darstellen, und die Alkylreste in $R^7$ und $R^8$ geradkettiges $C_1$-$C_7$-Alkyl bedeuten; m für 0 oder 1 steht; eine der Gruppen $X^3$ und $X^4$ eine Estergruppe -COO- oder -OOC- und die übrigen der Gruppen $X^3$, $X^4$, $X^5$ und $X^6$ eine einfache Kovalenzbindung bedeuten, oder eine dieser Gruppen auch die Gruppe $-CH_2CH_2-$ bedeutet; die Ringe $B^1$ und $B^5$ eine Gruppe der Formel

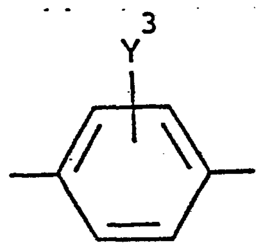

XXXII

oder trans-1,4-Cyclohexylen bezeichnen; die Ringe $B^2$, $B^3$ und $B^4$ eine Gruppe der Formel XXXII oder, sofern sie nicht mit mindestens einem der beiden andern dieser Ringe durch eine einfache Kovalenzbindung verknüpft sind, auch trans-1,4-Cyclohexylen darstellen; $Y^3$ Wasserstoff oder an einem der Ringe der Formel XXXII, welcher nicht mit einem weiteren Ring über eine einfache Kovalenzbindung verknüpft ist, auch Fluor, Chlor oder Methyl bedeutet; $R^{11}$ und $R^{12}$ geradkettiges $C_1$-$C_7$-Alkyl oder an einem Ring der Formel XXXII auch geradkettiges $C_1$-$C_7$-Alkoxy bezeichnen.

Besonders bevorzugt sind die Verbindungen der Formeln XXI, XXII, XXVI, XXIX, XXX und XXXI.

Als besonders geeignete Verbindungen für Komponente B haben sich nun die Verbindungen der obigen Formel I erwiesen. Weitere, für Komponente B geeignete Verbindungen,

welche im Gemisch mit einer oder mehreren Verbindungen der Formel I verwendet werden können, sind beispielsweise die in Z. Chemie 17, 333 (1977), J. prakt. Chemie 151, 221 (1938), Z. Chemie 6, 467 (1966) und Mol. Cryst. Liq. Cryst. 25, 299 (1974) erwähnten Phenyl- und Diphenylpyridazine und die in den Deutschen Offenlegungsschriften 2 933 563 und 2 937 700 beschriebenen Verbindungen mit zwei lateralen Cyanogruppen. Besonders geeignete Verbindungen für Komponente B, welche im Gemisch mit einer oder mehreren Verbindungen der Formel I verwendet werden können, sind die Verbindungen der folgenden allgemeinen Formeln:

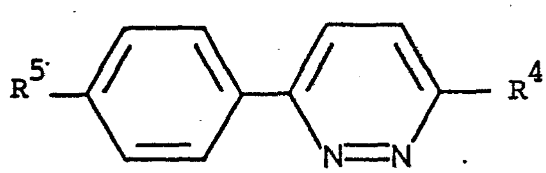
XXXIII

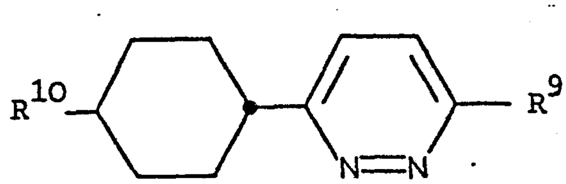
XXXIV

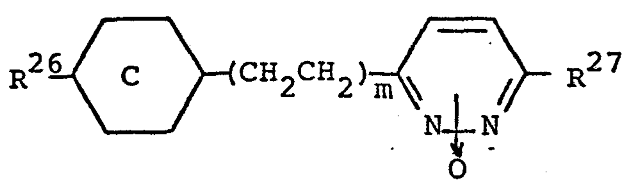
XX

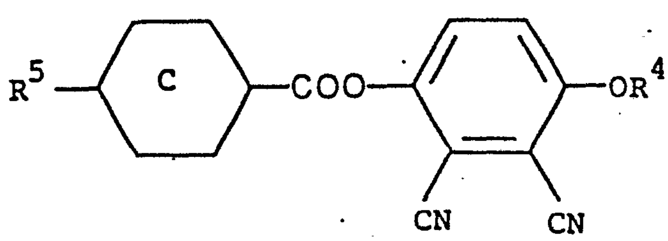
XXXV

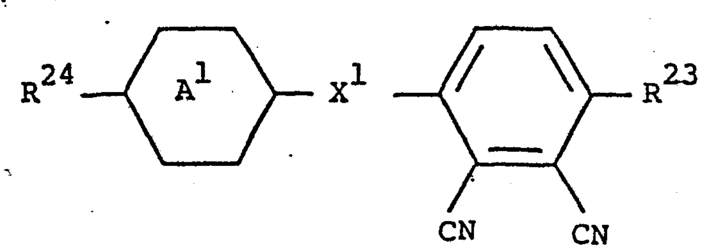
XXXVI

worin $R^4$ und $R^5$ geradkettiges $C_1$-$C_8$-Alkyl und Ring C 1,4-Phenylen oder trans-1,4-Cyclohexylen bezeichnen; $R^9$ geradkettiges $C_1$-$C_{10}$-Alkyl, $C_2$-$C_{10}$-1-Alkinyl, $C_1$-$C_{10}$-Alkoxy, p-($C_1$-$C_{10}$-Alkyl)phenyl, p-($C_1$-$C_{10}$-Alkoxy)phenyl oder trans-4-($C_1$-$C_{10}$-Alkyl)cyclohexyl und $R^{10}$ geradkettiges $C_1$-$C_{12}$-Alkyl bedeuten; $R^{23}$ und $R^{24}$ geradkettiges $C_1$-$C_{12}$-Alkyl oder an einem aromatischen Ring auch geradkettiges $C_1$-$C_{12}$-Alkoxy bedeuten, oder einer der Reste $R^{23}$ und $R^{24}$ auch eine Gruppe der allgemeinen Formel

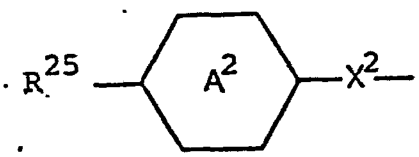

XXXVII

bedeutet; $X^1$ und $X^2$ für einfache Kovalenzbindungen oder eine dieser Gruppen auch für $-CH_2CH_2-$ stehen; die Ringe $A^1$ und $A^2$ 1,4-Phenylen oder, sofern $X^1$ oder $X^2$ für $-CH_2CH_2-$ steht, auch trans-1,4-Cyclohexylen darstellen; und $R^{25}$ geradkettiges $C_1$-$C_{12}$-Alkyl oder an einem aromatischen Ring $A^2$ auch geradkettiges $C_1$-$C_{12}$-Alkoxy bedeutet; m für die Zahl 0 oder 1 steht; $R^{26}$ $C_1$-$C_{12}$-Alkyl oder, sofern Ring C 1,4-Phenylen darstellt, auch $C_1$-$C_{12}$-Alkoxy bedeutet und $R^{27}$ $C_1$-$C_{12}$-Alkyl, $C_1$-$C_{12}$-Alkoxy oder $C_2$-$C_{12}$-1-Alkinyl bezeichnet; und die Alkyl-, Alkoxy- und 1-Alkinylgruppen in $R^9$, $R^{26}$ und $R^{27}$ geradkettige Reste bezeichnen.

Besonders bevorzugt sind die Verbindungen der Formeln XXXIV und XXXVI.

Geeignete Verbindungen für Komponente C sind beispielsweise die in Mol. Cryst. Liq. Cryst. 63, 129 (1981) und den Deutschen Offenlegungsschriften 2 736 772, 2 752 975 und 3 046 872 beschriebenen Verbindungen mit 3 oder 4 1,4-Phenylen- bzw. trans-1,4-Cyclohexylengruppen, einer polaren Endgruppe und gegebenenfalls einem lateralen Halogen- oder Cyanosubstituenten. Besonders geeignete

Verbindungen für Komponente C sind jedoch die Verbindungen der allgemeinen Formel

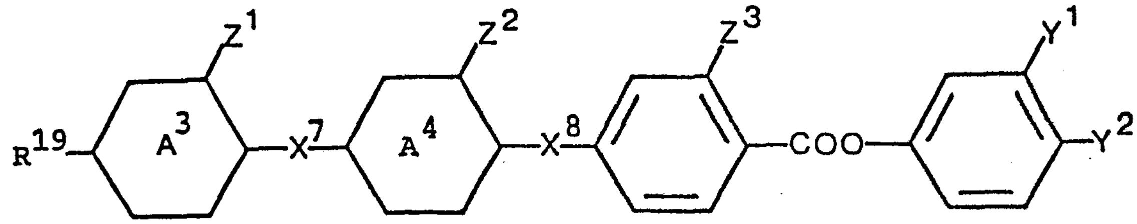

XXXVIII

worin $X^8$ eine einfache Kovalenzbindung oder die Estergruppe -COO- bezeichnet; $X^7$ eine einfache Kovalenzbindung, die Estergruppe -COO-, die Aethylengruppe $-CH_2CH_2-$ oder, sofern $X^8$ die Estergruppe -COO- bezeichnet, auch 1,4-Phenylen bedeutet; Ring $A^3$ für einen Benzolring oder für trans-1,4-Cyclohexylen steht; Ring $A^4$ einen Benzolring oder, sofern $X^8$ die Estergruppe -COO- und $X^7$ eine einfache Kovalenzbindung, die Estergruppe -COO- oder die Aethylengruppe $-CH_2CH_2-$ bezeichnet, auch trans-1,4-Cyclohexylen darstellt; die Reste $Z^1$, $Z^2$ und $Z^3$ Wasserstoff oder an einem Benzolring, welcher nicht mit einem weiteren Ring über eine einfache Kovalenzbindung direkt verknüpft ist, auch Halogen, Cyano oder Methyl bedeuten; $Y^2$ Cyano, Nitro, 2,2-Dicyanovinyl oder, sofern $Y^1$ für Wasserstoff steht, auch 2,2-Dicyano-1-methylvinyl darstellt; $Y^1$ Halogen, Cyano, $C_1$-$C_3$-Alkyl oder, sofern $X^7$ für p-Phenylen oder $Y^2$ für Nitro steht oder $Z^1$ und/oder $Z^2$ von Wasserstoff verschieden ist, auch Wasserstoff bezeichnet; und $R^{19}$ $C_1$-$C_{12}$-Alkyl oder an einem Benzolring auch $C_1$-$C_{12}$-Alkoxy bedeutet,

und die Verbindungen der allgemeinen Formel

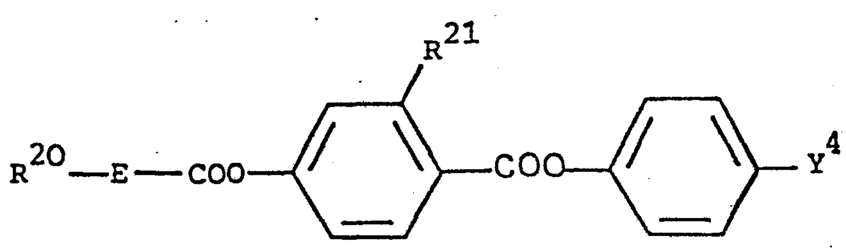

XXXIX

worin $R^{21}$ Wasserstoff, Fluor, Chlor, Brom oder die Cyanogruppe bezeichnet, $Y^4$ 2,2-Dicyanovinyl, 2,2-Dicyano-1-methylvinyl oder Cyano darstellt, $R^{20}$ und E zusammen p-$R^{20}$-Phenyl, trans-4-$R^{20}$-Cyclohexyl, 4'-$R^{20}$-4-Biphenylyl, p-(trans-4-$R^{20}$-Cyclohexyl)-phenyl, p-(5-$R^{20}$-2-Pyrimidinyl)phenyl, p-[2-(p'-$R^{20}$-Phenyl)äthyl]phenyl, p-[2-(trans-4-$R^{20}$-Cyclohexyl)-äthyl]phenyl, trans-4-[2-(p-$R^{20}$-Phenyl)äthyl]cyclohexyl oder trans-4-[2-(trans-4-$R^{20}$-Cyclohexyl)äthyl]-cyclohexyl bedeutet, und $R^{20}$ geradkettiges $C_1$-$C_{12}$-Alkyl oder an einem Benzolring auch geradkettiges $C_1$-$C_{12}$-Alkoxy bezeichnet.

Diese Verbindungen weisen grosse nematische Mesophasenbereiche, niedrige Cross-over-Frequenzen und grosse absolute dielektrische Anisotropien auf. Der obige Ausdruck "Halogen" steht für Fluor, Chlor oder Brom. Vorzugsweise bedeuten die lateralen Substituenten $Z^1$, $Z^2$, $Z^3$ und $R^{21}$ Wasserstoff oder Chlor.

Die erfindungsgemässen Mischungen mit negativer dielektrischer Anisotropie bestehen zweckmässig aus einem flüssigkristallinen Trägermaterial mit einer dielektrischen Anisotropie von höchstens etwa +1 und mindestens einer Verbindung der Formel I. Geeignete Trägermaterialien sind in grosser Zahl bekannt, und enthalten eine oder mehrere Verbindungen mit negativer oder kleiner positiver Anisotropie der Dielektrizitätskonstante (Verbindungen mit positiver dielektrischer Anisotropie dürfen bei der hier besprochenen Anwendung definitionsgemäss nur in Mengen verwendet werden, welche die Anisotropie der Gesamtmischung nicht positiv werden lassen). Beispiele besonders geeigneter Verbindungen sind die oben, im Zusammenhang mit Komponenten A und B genannten Verbindungen und insbesondere die Verbindungen der Formeln XXI, XXII, XXVI, XXIX, XXX, XXXI, XXXIV, XXXV und XXXVI.

Die erfindungsgemässen Mischungen können ebenfalls optisch aktive Verbindungen, beispielsweise optisch aktive

Biphenyle, und/oder dichroitische Farbstoffe, beispielsweise Azo-, Azoxy- oder Anthrachinonfarbstoffe, enthalten (im Falle der Mischungen für die Zwei-Frequenz-Adressierung je nach Eigenschaften als Bestandteile der Komponenten A, B und C und im Falle der Mischungen mit negativer dielektrischer Anisotropie als Bestandteil des Trägermaterials). Der Anteil solcher Verbindungen wird durch die Löslichkeit, die gewünschte Ganghöhe (pitch), Farbe, Extinktion und dergleichen bestimmt. Vorzugsweise beträgt der Anteil optisch aktiver Verbindungen höchstens etwa 4 Gew.-% und der Anteil dichroitischer Farbstoffe höchstens etwa 10 Gewichtsprozente im Gesamtgemisch.

Die Herstellung der erfindungsgemässen flüssigkristallinen Mischungen kann in an sich bekannter Weise erfolgen, z.B. durch Erhitzen einer Mischung der Bestandteile auf eine Temperatur knapp oberhalb des Klärpunktes und anschliessendes Abkühlen.

Die Herstellung einer elektro-optischen Vorrichtung enthaltend eine erfindungsgemässe Mischung kann ebenfalls in an sich bekannter Weise erfolgen, z.B. durch Evakuieren einer geeigneten Zelle und Einbringen der Mischung in die evakuierte Zelle.

Die Verbindungen der Formel XXX sind neu. Sie können anhand der folgenden Reaktionsschemata 1 und 2 hergestellt werden, worin $R^{15}$ trans-4-Alkylcyclohexyl, 4'-Alkyl-4-biphenylyl, p-(trans-4-Alkylcyclohexyl)phenyl oder 2-(trans-4-Alkylcyclohexyl)äthyl und $R^{16}$ trans-4-Alkylcyclohexyl darstellen, oder $R^{15}$ trans-4-Alkylcyclohexyl und $R^{16}$ p-(trans-4-Alkylcyclohexyl)phenyl, p-[2-(trans-4-Alkylcyclohexyl)äthyl]phenyl oder 4'-(trans-4-Alkylcyclohexyl)-4-biphenylyl darstellen, oder $R^{15}$ p-Alkylphenyl und $R^{16}$ p-[2-(trans-4-Alkylcyclohexyl)äthyl]phenyl darstellen, und $R^{13}$ und $R^{14}$ sowie die Alkylgruppen in den Substituenten $R^{15}$ und $R^{16}$ geradkettiges $C_1$-$C_7$-Alkyl bezeichnen.

Schema 1

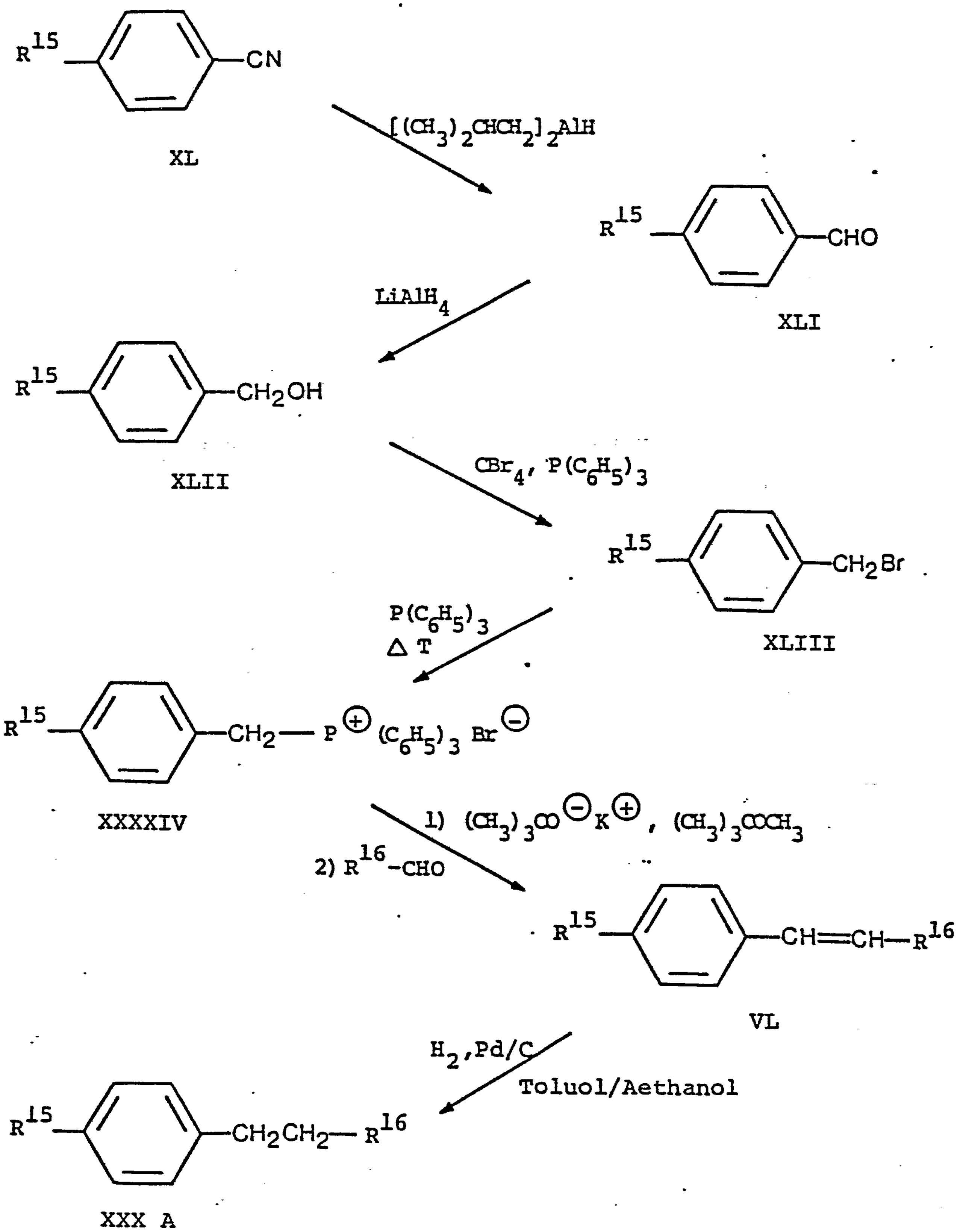
XL
[(CH3)2CHCH2]2AlH
XLI
LiAlH4
XLII
CBr4, P(C6H5)3
XLIII
P(C6H5)3
Δ T
XXXXIV
1) (CH3)3CO⊖K⊕, (CH3)3COCH3
2) R16-CHO
VL
H2,Pd/C
Toluol/Aethanol
XXX A

Schema 2

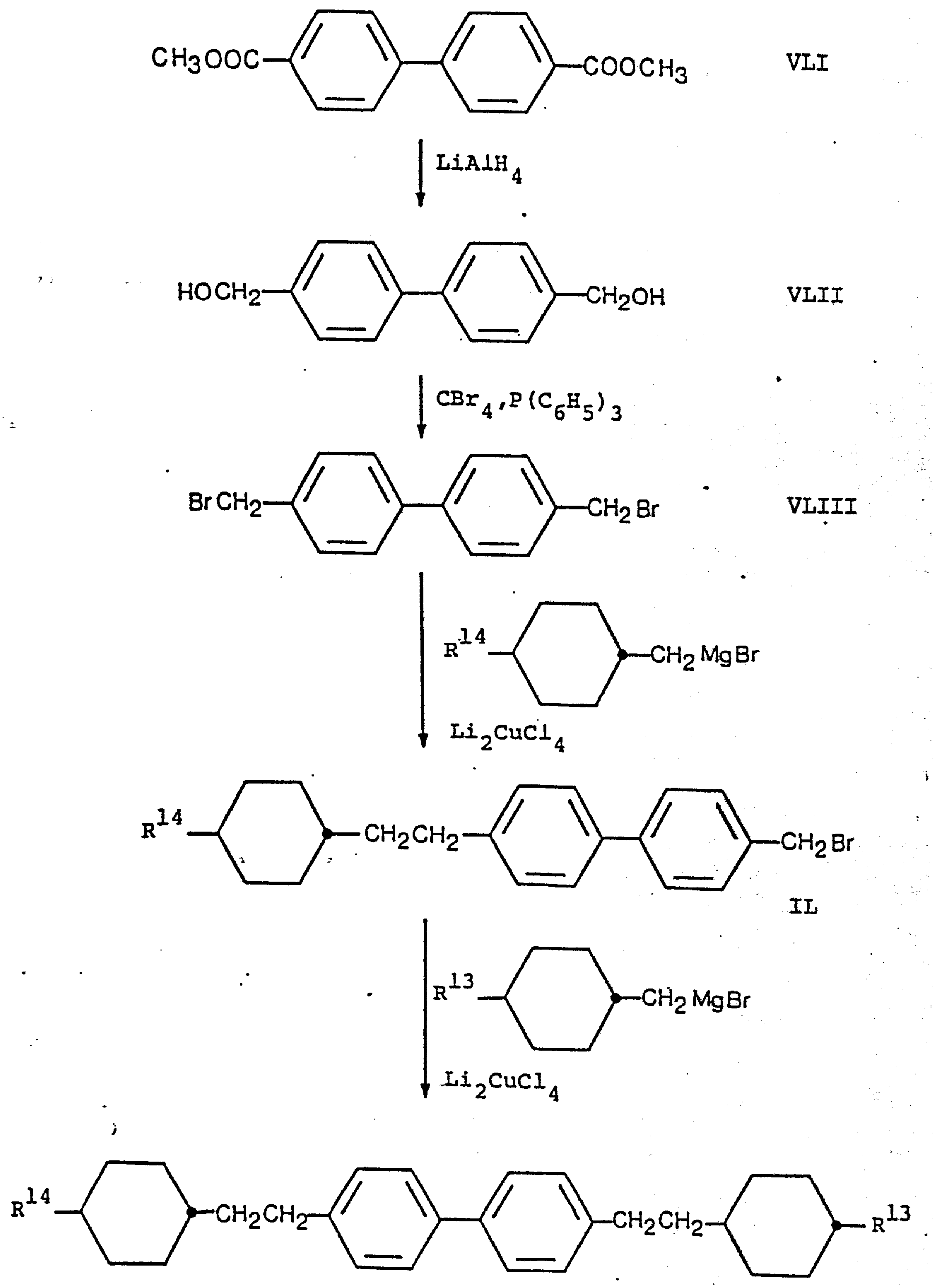


VLI

VLII

VLIII

IL

XXX B

Die Verbindungen der Formel $R^{16}$ -CHO in Schema 1 können in einfacher Weise aus bekannten Verbindungen erhalten werden, z.B. die trans-4-Alkylcyclohexancarboxaldehyde durch Rosenmund-Reduktion der entsprechenden Säurechloride und die übrigen Verbindungen durch Reduktion der entsprechenden Cyanoverbindungen.

Durch Umsetzung der Verbindung der Formel VLIII mit Grignard-Reagenzien gemäss Schema 2 können Verbindungen der Formel IL oder direkt Verbindungen der Formel XXXB, worin $R^{13}$ und $R^{14}$ gleiche Bedeutung haben, erhalten werden. Bei Verwendung von mindestens etwa 2 Mol Grignard-Reagens pro Mol der Verbindung der Formel VLIII wird im allgemeinen vorwiegend direkt eine Verbindung der Formel XXXB gebildet.

Die Ester der Formel XXXI sind ebenfalls neu. Sie können nach an sich bekannten Veresterungsmethoden (z.B. in analoger Weise zur unten beschriebenen Herstellung der Verbindungen der Formel XXXVIII) erhalten werden. Die hierbei benötigten Ausgangsmaterialien sind bekannte oder Analoge bekannter Verbindungen und können nach bekannten Methoden hergestellt werden.

Die Verbindungen der Formel XXXIV sind ebenfalls neu. Sie können in an sich bekannter Weise dadurch hergestellt werden, dass man

a) zur Herstellung der Verbindungen der Formel XXXIV, worin $R^9$ eine Alkyl-, p-Alkylphenyl, p-Alkoxyphenyl oder trans-4-Alkylcyclohexylgruppe darstellt, eine Verbindung der allgemeinen Formel

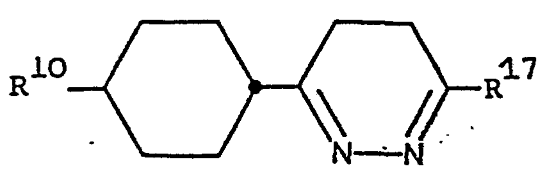

L

worin $R^{17}$ eine Alkyl-, p-Alkylphenyl, p-Alkoxyphenyl oder trans-4-Alkylcyclohexylgruppe darstellt, die Alkyl- und Alkoxyreste in $R^{17}$ geradkettige Reste mit 1 bis 10 Kohlenstoffatomen sind, und $R^{10}$ die obige Bedeutung hat,

oder ein tautomeres Dihydropyridazin oxidiert (z.B. mit 2,3-Dichlor-5,6-dicyano-p-benzochinon in Dioxan, mit Natriumnitrit in Eisessig und Aethanol, mit Isopentylnitrit in Eisessig oder vorzugsweise durch katalytische Dehydrierung mit Palladium, Platin und dergleichen), oder

b) zur Herstellung der Verbindungen der Formel XXXIV, worin $R^9$ eine Alkoxygruppe darstellt, eine Verbindung der allgemeinen Formel

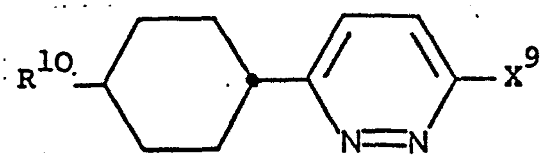 LI

worin $X^9$ Chlor oder Brom bezeichnet und $R^{10}$ die obige Bedeutung hat,

mit einem Alkalimetallalkoholat (z.B. Natriumalkanolat) umsetzt, oder

c) zur Herstellung der Verbindungen der Formel XXXIV, worin $R^9$ die Aethinylgruppe darstellt, eine Verbindung der allgemeinen Formel

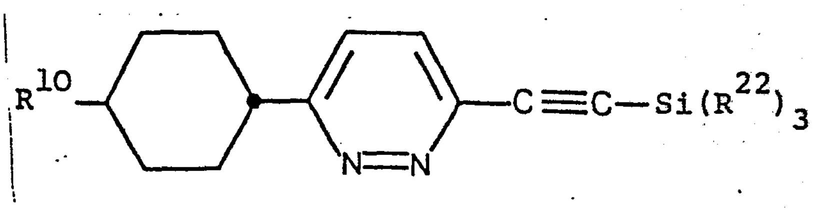 LII

worin $R^{22}$ eine Alkylgruppe mit 1 bis 5 Kohlenstoffatomen bezeichnet und $R^{10}$ die obige Bedeutung hat,

mit Base (z.B. Kaliumhydroxid, Natriumhydroxid oder Butyllithium) umsetzt, oder

d) zur Herstellung der Verbindungen der Formel XXXIV, worin $R^9$ eine 1-Alkinylgruppe mit 3 bis 10 Kohlenstoffatomen darstellt, eine Verbindung der allgemeinen Formel

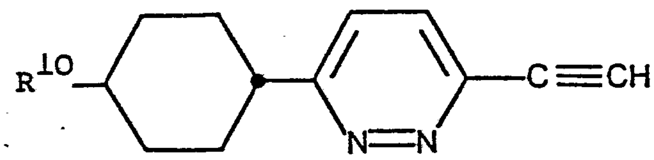

LIII

worin $R^{10}$ die obige Bedeutung hat, mit Base (z.B. Butyllithium, Methyllithium, Natriumamid oder Lithiumdiisopropylamid) in das entsprechende Aethinylid überführt und mit Alkylbromid oder -jodid alkyliert.

Die Verbindungen der Formel L können durch Wanderung der Doppelbindungen im Dihydropyridazin-Ring zu tautomeren Verbindungen umlagern. Derartige Umlagerungen können z.B. durch Anwesenheit einer Spur Säure oder Base ausgelöst werden. Da die tautomeren Dihydropyridazine jedoch ebenfalls unter den obigen Bedingungen zu Verbindungen der Formel XXXIV oxidiert werden können, kann gemäss Verfahrensvariante a) sowohl eine Verbindung der Formel L, als auch ein tautomeres Dihydropyridazin oder ein Gemisch solcher Verbindungen umgesetzt werden.

Die Ausgangsmaterialien der Formeln L und LI sind neu. Sie können anhand der folgenden Reaktionsschemata 3-6 hergestellt werden, worin $R^{10}$, $R^{17}$ und $X^9$ die obigen Bedeutungen haben und $R^{18}$ eine geradkettige Alkyl- oder Alkoxygruppe mit 1 bis 10 Kohlenstoffatomen darstellt.

Schema 3

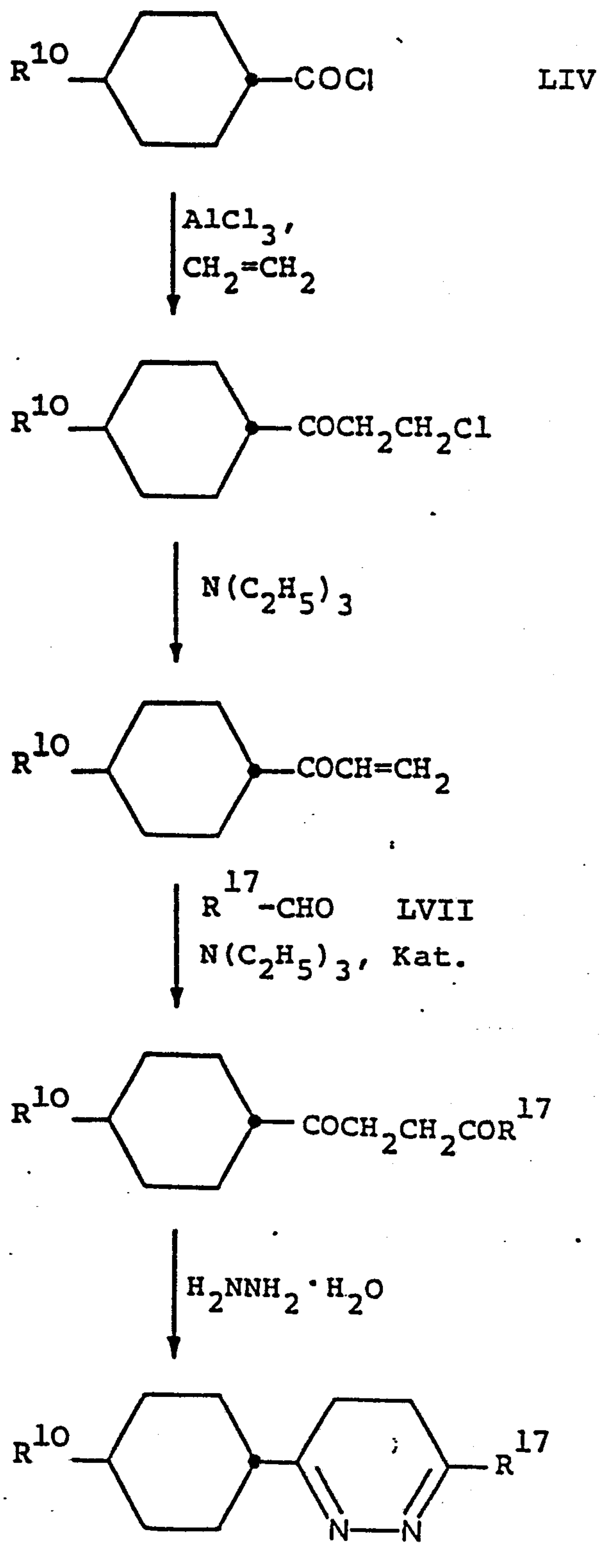


LIV

LV

LVI

LVIII

L

Schema 4

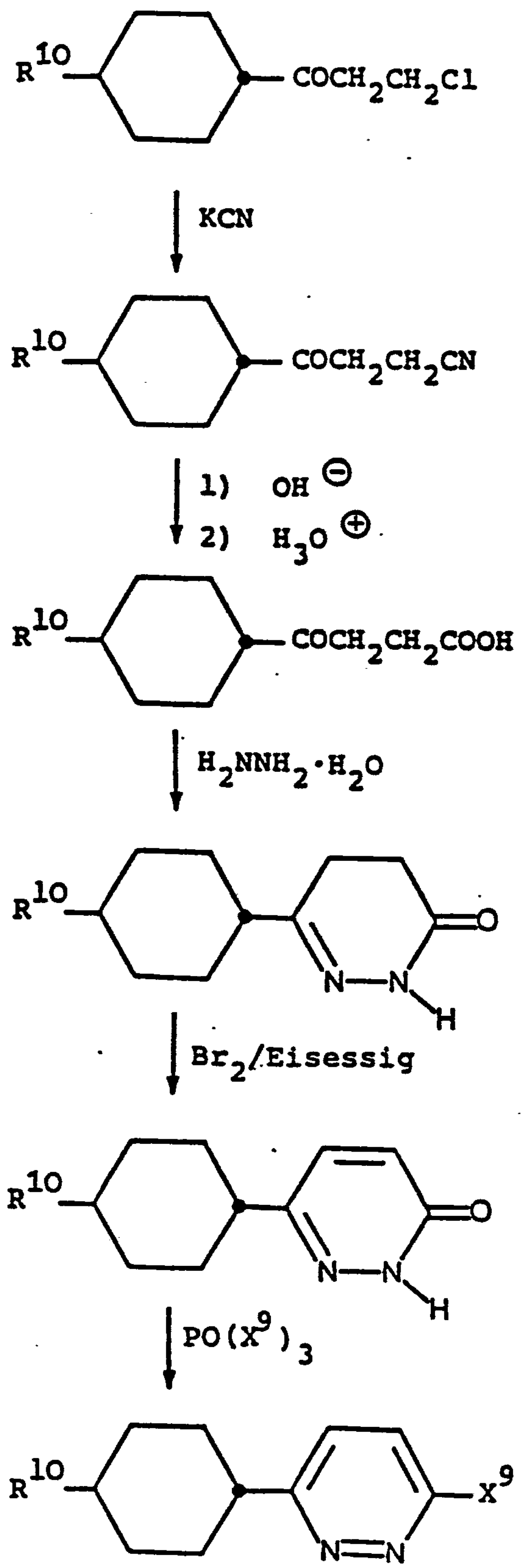


LV

LIX

LX

LXI

LXII

LI

Schema 5

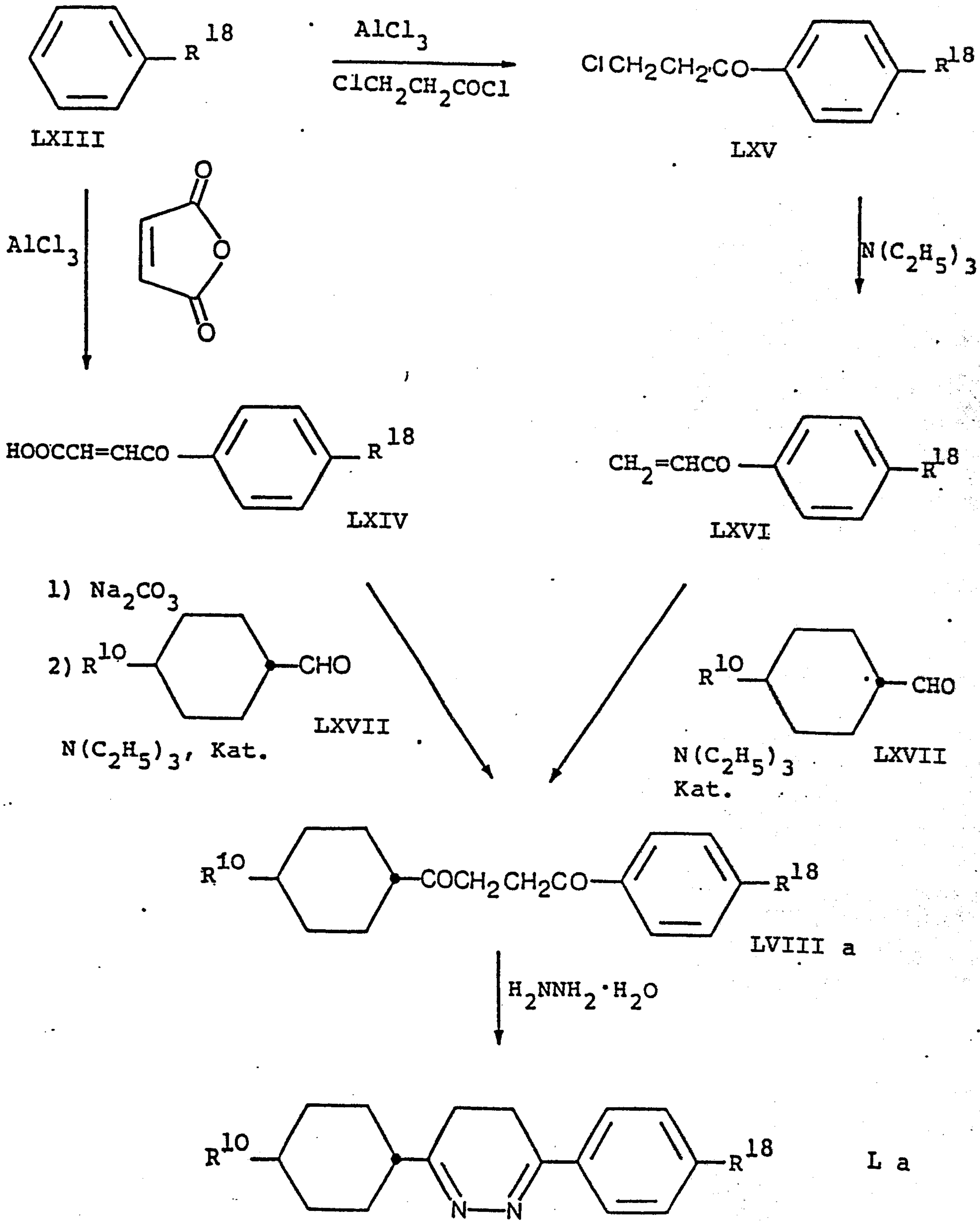
AlCl3
ClCH2CH2COCl
LXIII
LXV
AlCl3
N(C2H5)3
LXIV
LXVI
1) Na2CO3
2)
LXVII
N(C2H5)3, Kat.
N(C2H5)3
Kat.
LXVII
LVIII a
H2NNH2·H2O
L a

Schema 6

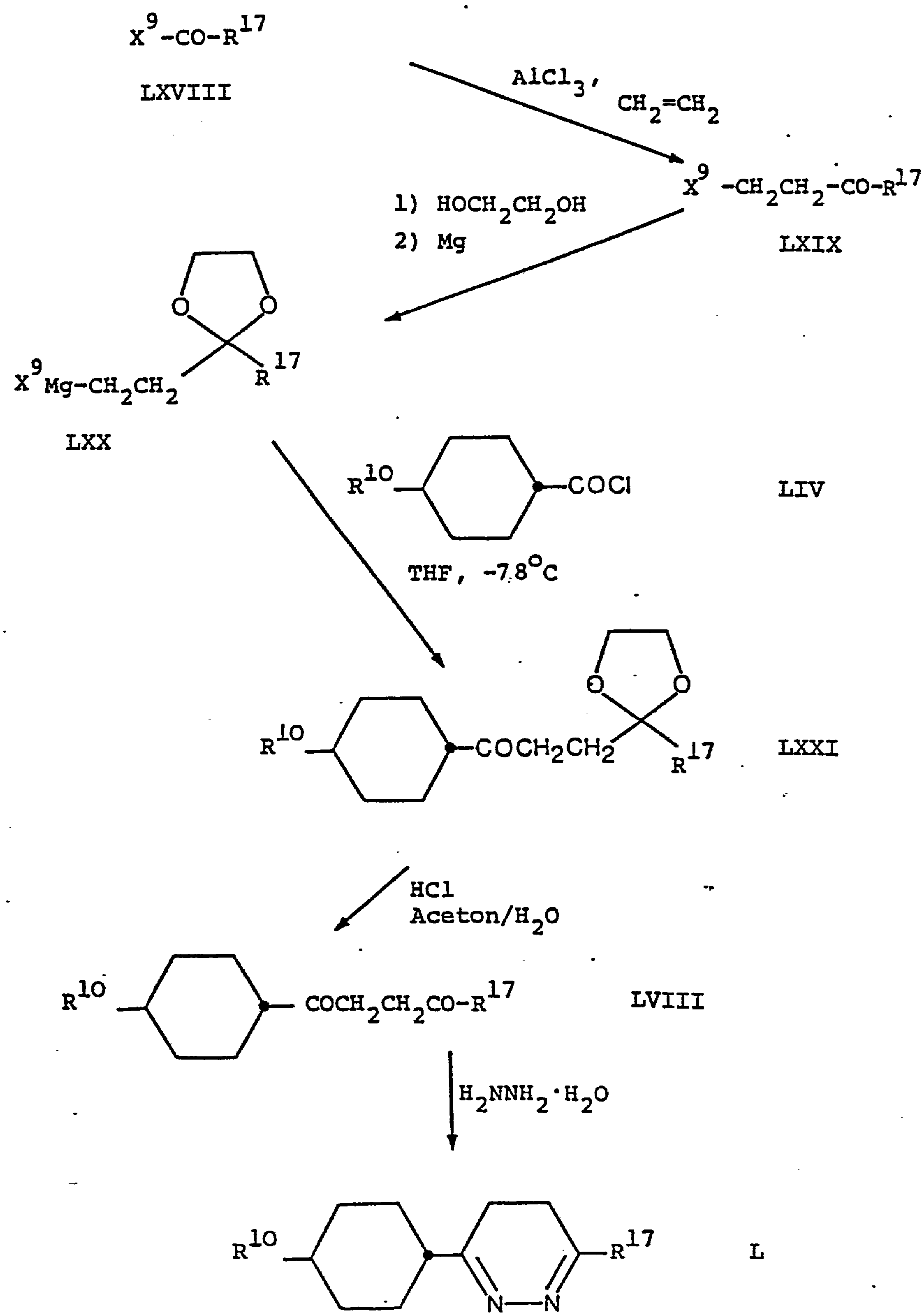
LXVIII
$AlCl_3$, $CH_2=CH_2$
LXIX
1) $HOCH_2CH_2OH$
2) Mg
LXX
LIV
THF, -78°C
LXXI
HCl
Aceton/$H_2O$
LVIII
$H_2NNH_2 \cdot H_2O$
L

Die Ausgangssubstanzen der Formeln LIV, LVII, LXIII, LXVII und LXVIII sind bekannte oder Analoge bekannter Verbindungen und können in bekannter Weise hergestellt werden. Beispielsweise können die Aldehyde der Formel LXVII durch Rosenmund-Reduktion der Säurechloride der Formel LIV hergestellt werden.

Die Addition eines Aldehydes an eine Verbindung der Formel LVI, LXIV oder LXVI kann nach der Methode von Stetter (Chem. Ber. 114 (1981) 581) in Gegenwart eines 1,3-Thiazoliumsalz-Katalysators durchgeführt werden. Bevorzugter Katalysator für die Addition eines Aldehydes der Formel LXVII bzw. eines Aldehyds der Formel LVII, worin $R^{17}$ Alkyl oder trans-4-Alkylcyclohexyl bedeutet, ist 3-Benzyl-5-(2-hydroxyäthyl)-4-methyl-1,3-thiazoliumchlorid und für die Addition eines Aldehyds der Formel LVII, worin $R^{17}$ p-Alkylphenyl oder p-Alkoxyphenyl bedeutet, 3,4-Dimethyl-5-(2-hydroxyäthyl)-1,3-thiazoliumjodid.

Die Kopplung einer Verbindung der Formel LXX mit einer Verbindung der Formel LIV kann nach der von T. Sato et al. in Bull. Chem. Soc. Japan 54 (1981) 505 beschriebenen Methode erfolgen.

Die Verbindungen der Formel LII sind ebenfalls neu. Sie können in an sich bekannter Weise dadurch erhalten werden, dass man eine Verbindung der Formel LI in Gegenwart von Triäthylamin, Bis-(triphenylphosphin)-palladium-(II)-dichlorid und Kupfer-(I)-jodid mit Aethinyltrialkylsilan umsetzt.

Die Verbindungen der Formel XX sind ebenfalls neu. Sie können in an sich bekannter Weise dadurch hergestellt werden, dass man

a) eine Verbindung der allgemeinen Formel

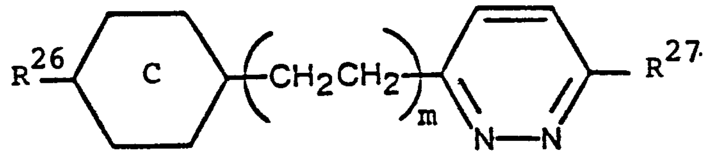

CV

worin m, C, $R^{26}$ und $R^{27}$ die in Formel XX gegebenen Bedeutungen haben,

mit Persäure oxidiert, oder

b) zur Herstellung der Verbindungen der Formel XX, worin $R^{27}$ $C_2$-$C_{12}$-1-Alkinyl bedeutet, eine Verbindung der allgemeinen Formel

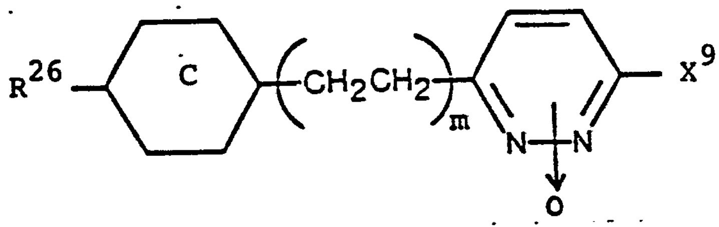

CVI

worin $X^9$ Chlor oder Brom bezeichnet und m, C und $R^{26}$ die in Formel XX gegebenen Bedeutungen haben,

alkinyliert (in analoger Weise zu Chem. Pharm. Bull. 28, 3488 (1980) und der obigen Verfahrensvariante a) bei der Herstellung der Verbindungen der Formel I).

Die Verbindungen der Formel XXXVI sind ebenfalls neu. Sie können in an sich bekannter Weise dadurch hergestellt werden, dass man

a) zur Herstellung der Verbindungen der Formel XXXVI, worin $R^{23}$ geradkettiges $C_1$-$C_{12}$-Alkyl oder eine Gruppe der Formel XXXVII bedeutet, eine Verbindung der allgemeinen Formel

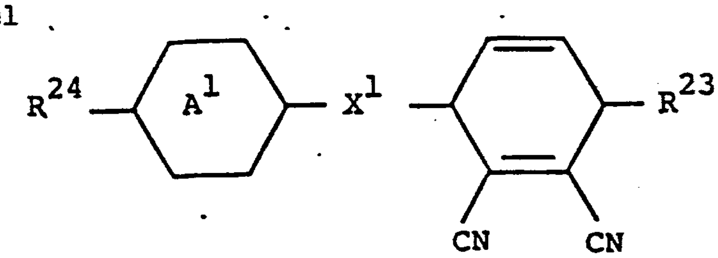

LXXII

worin $R^{23}$ geradkettiges $C_1$-$C_{12}$-Alkyl oder eine Gruppe der Formel XXXVII darstellt und $R^{24}$, $X^1$ und Ring $A^1$ die in Formel XXXVI gegebenen Bedeutungen haben,

oxidiert (z.B. mit 2,3-Dichloro-5,6-dicyano-p-benzochinon in Dioxan), oder

b) zur Herstellung der Verbindungen der Formel XXXVI, worin $R^{23}$ geradkettiges $C_1$-$C_{12}$-Alkoxy bedeutet, eine Verbindung der allgemeinen Formel

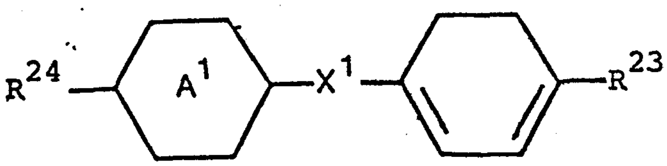
LXXIII

worin $R^{23}$ geradkettiges $C_1$-$C_{12}$-Alkoxy darstellt und $R^{24}$, $X^1$ und Ring $A^1$ die in Formel XXXVI gegebenen Bedeutungen haben,

in einem Aether (z.B. Tetrahydrofuran) mit Dicyanoacetylen umsetzt und anschliessend durch Erhitzen Aethylen abspaltet.

Die Verbindungen der Formel LXXII können beispielsweise dadurch hergestellt werden, dass man einen Aldehyd der allgemeinen Formel

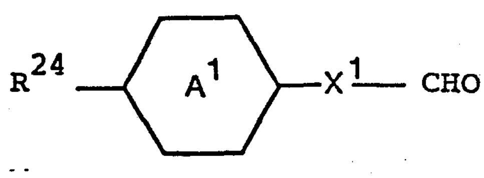
LXXIV

mit einem Phosphoniumsalz der allgemeinen Formel

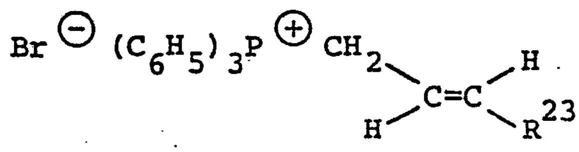
LXXV

worin $R^{23}$, $R^{24}$, $X^1$ und Ring $A^1$ die in Formel LXXII gegebenen Bedeutungen haben,

in Diäthyläther in Gegenwart von Butyllithium umsetzt und das erhaltene Dien durch Diels-Alder-Reaktion mit Dicyanoacetylen in Tetrahydrofuran in eine Verbindung der Formel LXXII überführt.

Die Verbindungen der Formel LXXIII können beispielsweise dadurch erhalten werden, dass man eine Verbindung

der allgemeinen Formel

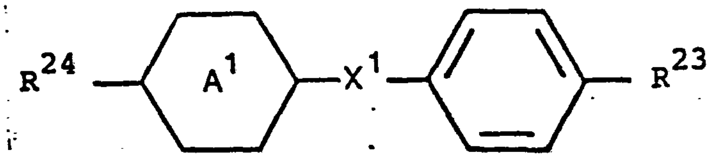 LXXVI

worin $R^{23}$, $R^{24}$, $X^1$ und Ring $A^1$ die in Formel LXXIII gegebenen Bedeutungen haben,

mit Lithium und flüssigem Ammoniak (vorzugsweise in einem Diäthyläther/Aethanol-Gemisch) reduziert. Hierbei wird im allgemeinen das 1,4-Dien oder ein Gemisch des 1,3-Diens (Verbindung der Formel LXXIII) und des 1,4-Diens erhalten. Die Isomerisierung zum 1,3-Dien kann beispielsweise mit 2,3-Dichlormaleinsäureanhydrid erfolgen.

Die Verbindungen der Formel XXXVIII sind ebenfalls neu. Sie können in an sich bekannter Weise dadurch hergestellt werden, dass das Säurechlorid einer Verbindung der allgemeinen Formel

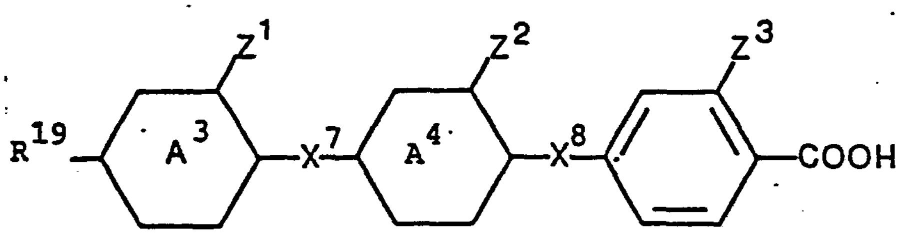 LXXVII

worin $R^{19}$, $A^3$, $A^4$, $X^7$, $X^8$, $Z^1$, $Z^2$ und $Z^3$ die in Formel XXXVIII gegebenen Bedeutungen haben,

mit einem Phenol der allgemeinen Formel

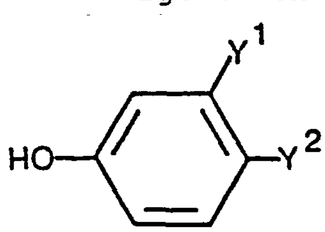 LXXVIII

worin $Y^1$ und $Y^2$ die in Formel XXXVIII gegebene Bedeutung haben,

verestert und, gewünschtenfalls, eine erhaltene Verbindung der Formel XXXVIII, worin $Z^1$, $Z^2$ oder $Z^3$ Brom bedeutet, mit Kupfer-(I)-, Natrium- oder Kaliumcyanid umsetzt.

Die Verbindungen der Formel LXXVIII, worin $Y^2$ 2,2-Dicyanovinyl bedeutet, können beispielsweise dadurch hergestellt werden, dass man 3-$Y^1$-Anisol durch Vilsmeier-Reaktion mit Dimethylformamid in Gegenwart von Phosphoroxychlorid zu 4-Methoxy-2-$Y^1$-benzaldehyd umsetzt, dann die Methoxygruppe hydrolysiert (z.B. durch Erhitzen unter Rückfluss mit Pyridiniumchlorid und anschliessende fraktionierte Destillation) und schliesslich den erhaltenen 4-Hydroxy-2-$Y^1$-benzaldehyd durch Knoevenagel-Kondensation mit Malonitril (z.B. in Gegenwart katalytischer Mengen Eisessig und Natriumacetat in siedendem Toluol) in die Verbindung der Formel LXXVIII, worin $Y^2$ 2,2-Dicyanovinyl bedeutet überführt. Die übrigen Verbindungen der Formel LXXVIII sind bekannte oder Analoge bekannter Verbindungen.

Die Verbindungen der Formel LXXVII sind ebenfalls bekannte oder Analoge bekannter Verbindungen und können nach bekannten Methoden hergestellt werden.

Die Verbindungen der Formel LXXVII, worin $X^8$ die Estergruppe -COO- bezeichnet können beispielsweise dadurch hergestellt werden, dass man eine Verbindung der allgemeinen Formel

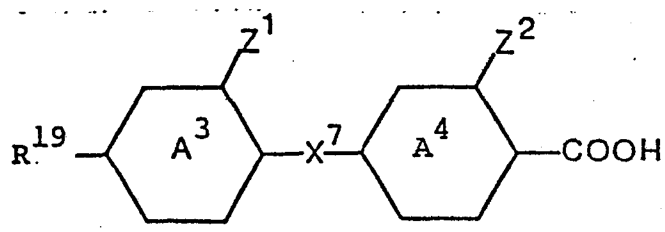

LXXIX

worin $X^7$ eine einfache Kovalenzbindung, die Estergruppe -COO-, die Aethylengruppe $-CH_2CH_2-$ oder 1,4-Phenylen bezeichnet und $R^{19}$, $A^3$, $A^4$, $Z^1$ und $Z^2$ die in Formel XXXVIII gegebenen Bedeutungen haben, in Methylenchlorid in Gegenwart von Dicyclohexylcarbodiimid und 4-(Dimethylamino)pyridin mit 4-Hydroxy-2-$Z^3$-benzaldehyd verestert und den erhaltenen Aldehyd durch Jones-Oxidation mit Chromsäure und Schwefelsäure in die entsprechende Säure der Formel LXXVII überführt.

Bei der Herstellung der Säuren der Formel LXXVII, worin $X^7$ die Aethylengruppe $-CH_2CH_2-$ und $X^8$ eine einfache Kovalenzbindung bedeuten, und der Säuren der Formel LXXIX, worin $X^7$ die Aethylengruppe $-CH_2CH_2-$ bedeutet, erfolgt die Verknüpfung der Ringe $A^3$ und $A^4$ zweckmässig durch Fouquet-Schlosser-Reaktion oder durch Wittig-Reaktion. Beispielsweise kann 4-(Brommethyl)-2-$Z^2$-benzonitril, 4'-(Brommethyl)-4-biphenylcarbonitril oder trans-4-(Tosyloxymethyl)cyclohexancarbonitril in Gegenwart von Dilithiumtetrachlorkuprat mit (4-$R^{19}$-2-$Z^1$-Phenyl)methylmagnesiumbromid bzw. (trans-4-$R^{19}$-Cyclohexyl)methylmagnesiumbromid umgesetzt und das erhaltene Nitril zur gewünschten Säure hydrolysiert werden. Ferner kann beispielsweise 4-$R^{19}$-2-$Z^1$-Benzaldehyd oder trans-4-$R^{19}$-Cyclohexancarboxaldehyd in Gegenwart einer Base (z.B. Natriummethylat) mit (4-Methoxycarbonyl-3-$Z^2$-phenyl)methyl-triphenylphosphoniumbromid (wobei $Z^1$ und $Z^2$ Wasserstoff, Fluor, Cyano oder Methyl bedeuten) umgesetzt, dann die Doppelbindung katalytisch hydriert und schliesslich die Estergruppe verseift werden.

Die für diese Reaktionen benötigten Ausgangsmaterialien sind bekannt oder können nach an sich bekannten Methoden hergestellt werden. Beispielsweise kann 4-Alkoxy-2-$Z^1$-acetophenon durch Haloformabbau zu 4-Alkoxy-2-$Z^1$-benzoesäure umgesetzt und diese durch Reduktion mit Lithiumaluminiumhydrid und Bromierung (z.B. mit Tetrabrommethan und Triphenylphosphin) in 4-Alkoxy-1-(brommethyl)-2-$Z^1$-benzol übergeführt werden. Aus 2,4-Dimethylbenzoesäuremethylester kann beispielsweise durch Umsetzung mit N-Bromsuccinimid und anschliessende Isomerentrennung 4-(Brommethyl)-2-methylbenzoesäure-methylester erhalten werden, welcher in analoger Weise zu Org. Synth. Coll. V, 825 zu 4-Formyl-2-methylbenzoesäure-methylester umgesetzt werden kann; der nach Umsetzung mit Alkyl-triphenylphosphoniumbromid und Base und anschliessender katalytischer Hydrierung der Doppelbindung erhaltene 4-Alkyl-2-methylbenzoesäure-methylester kann dann mit Natronlauge zur Säure verseift oder mit Lithiumaluminiumhydrid zum Alkohol

reduziert werden, welcher schliesslich mit Bromwasserstoff zu 4-Alkyl-1-(brommethyl)-2-methylbenzol oder mit Braunstein zum 4-Alkyl-2-methylbenzaldehyd weiter umgesetzt werden kann. 1-Alkyl-3-fluorbenzol kann beispielsweise durch Umsetzung mit Butyllithium und Kohlendioxid und anschliessende Hydrolyse in 4-Alkyl-2-fluorbenzoesäure übergeführt werden und 1-Alkyl-3-chlorbenzol bzw. 1-Alkyl-3-brombenzol kann durch Friedel-Crafts-Acylierung mit Acetylchlorid in Gegenwart von Aluminiumtrichlorid und anschliessende Oxidation mit Natriumhypobromit in 4-Alkyl-2-(chlor oder brom)benzoesäure übergeführt werden; die erhaltenen Säuren können dann mit Lithiumaluminiumhydrid zum Alkohol und dieser mit Bromwasserstoff zum Bromid oder mit Braunstein zum Aldehyd weiter umgesetzt werden. Ferner kann beispielsweise 4-Methyl-2-$Z^1$-benzoesäure der Reihe nach mit Thionylchlorid, Ammoniak und Benzolsulfonylchlorid umgesetzt und das erhaltene 4-Methyl-2-$Z^1$-benzonitril mit N-Bromsuccinimid zu 4-(Brommethyl)-2-$Z^1$-benzonitril weiter umgesetzt werden.

Die Verbindungen der Formel XXXIX sind zum Teil ebenfalls neue Verbindungen. Sie können in analoger Weise zu den Verbindungen der Formel XXXVIII durch Veresterung hergestellt werden. Die hierbei benötigten Säuren können anhand des Reaktionsschemas 7 erhalten werden, worin $R^{20}$, $R^{21}$ und E die in obiger Formel XXXIX gegebenen Bedeutungen haben:

Schema 7

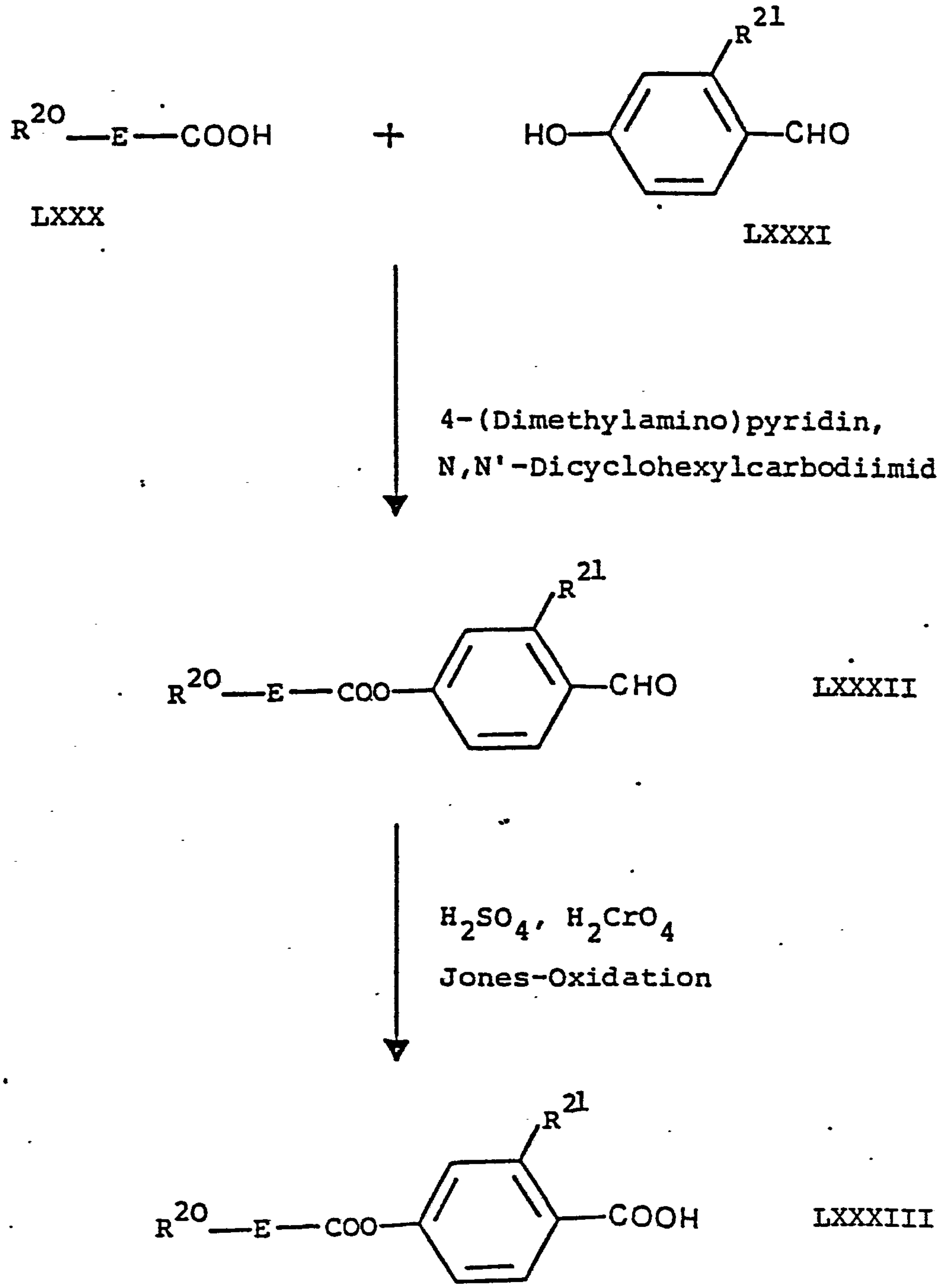


LXXX

LXXXI

LXXXII

LXXXIII

Die Verbindungen der Formeln LXXX und LXXXI sind bekannt oder können nach an sich bekannten Methoden hergestellt werden.

Die Erfindung betrifft ferner alle neuen Verbindungen, Mischungen, Verfahren, Verwendungen und Vorrichtungen wie hierin beschrieben.

Die Herstellung der Verbindungen der Formel I wird durch die folgenden Beispiele weiter veranschaulicht. C bedeutet eine kristalline, S eine smektische, N eine nematische und I die isotrope Phase.

Beispiel 1

In einem Sulfierkolben werden unter Stickstoffbegasung 0,51 g Natrium vorgelegt und dann langsam 13 ml trockenes Butanol so zugetropft, dass der Alkohol schwach siedet (Wasserstoffentwicklung). Zuletzt wird bis zur vollständigen Lösung des Natriums auf 60°C erwärmt. Nach dem Abkühlen auf 30°C wird innert 15 Minuten eine Lösung von 5,6 g 3-Chlor-6-[2-(trans-4-butylcyclohexyl)äthyl]pyridazin in 40 ml trockenem Butanol zugetropft. Anschliessend wird noch 4 Stunden bei 60°C gerührt, auf Raumtemperatur abgekühlt und das Lösungsmittel am Rotationsverdampfer entfernt. Der Rückstand wird in 100 ml Hexan aufgenommen und dreimal mit je 100 ml Wasser neutral gewaschen. Die wässrigen Phasen werden mit 100 ml Hexan nachextrahiert. Die organischen Phasen werden über Natriumsulfat getrocknet und am Rotationsverdampfer vom Lösungsmittel befreit. Das Rohprodukt wird an Aluminiumoxid (neutral) mit Hexan/Essigester chromatographiert und dann mehrmals aus Essigester und Hexan umkristallisiert. Hierbei wird reines 3-Butyloxy-6-[2-(trans-4-butylcyclohexyl)-äthyl]pyridazin erhalten.

Das als Ausgangsmaterial benötigte 3-Chlor-6-[2-(trans-4-butylcyclohexyl)äthyl]pyridazin kann wie folgt hergestellt werden:

a) 33,6 g trans-4-Butylcyclohexancarboxaldehyd und 128,8 g 1,3-Dioxolan-2-yl-methyl-triphenylphosphonium-bromid werden unter Stickstoffbegasung in 800 ml trockenem N,N-Dimethylformamid gelöst, auf 80-90°C erwärmt und bei dieser Temperatur innert 3 Stunden tropfenweise mit einer frisch zubereiteten Lösung von 2,1 g Lithium in 300 ml trockenem Methanol versetzt. Das Reaktionsgemisch wird noch 5 Stunden bei 80-90°C gerührt, dann auf Raumtemperatur abgekühlt und am Rotationsverdampfer eingeengt. Der Rückstand wird mit 800 ml Methylenchlorid verdünnt und zweimal mit je 400 ml Wasser gewaschen. Die wässrigen

Phasen werden mit 100 ml Methylenchlorid nachextrahiert. Die vereinigten organischen Phasen werden über Natriumsulfat getrocknet und eingeengt. Zur Entfernung des Triphenylphosphinoxids wird das erhaltene Rohprodukt von trans-4-Butyl-1-[2-(1,3-dioxolan-2-yl)vinyl]cyclohexan mit Methylenchlorid/Hexan an Kieselgel chromatographiert und dann das Lösungsmittel entfernt.

b) Das erhaltene rohe trans-4-Butyl-1-[2-(1,3-dioxolan-2-yl)vinyl]cyclohexan wird in 500 ml Aethanol gelöst, mit 1 ml Triäthylamin und 1 g Palladium/Kohle (5%) versetzt und unter Schütteln hydriert. Nach beendeter Wasserstoffaufnahme wird das Reaktionsgemisch filtriert und der Rückstand mit 100 ml Aethanol nachgewaschen. Das Filtrat wird am Rotationsverdampfer auf das halbe Volumen eingeengt, mit 300 ml Wasser und 5 ml konzentrierter Schwefelsäure versetzt und 30 Minuten auf 80°C erwärmt. Zur Aufarbeitung wird mit Wasser verdünnt, mit Natriumhydrogencarbonat neutralisiert und zweimal mit je 250 ml tert.-Butylmethyläther extrahiert. Die organischen Phasen werden über Natriumsulfat getrocknet und am Rotationsverdampfer vom Lösungsmittel befreit. Das erhaltene Rohprodukt von 3-(trans-4-Butylcyclohexyl)propanal wird im Hochvakuum destilliert.

c) Unter Stickstoffbegasung werden 7,5 g Acrylsäureäthylester in 100 ml trockenem Dioxan gelöst, mit 6,1 g Triäthylamin und 1,4 g 3-(2-Aethoxyäthyl)-5-(2-hydroxyäthyl)-4-methyl-1,3-thiazoliumbromid versetzt und auf 80°C erwärmt. Innert 5 Stunden wird eine Lösung von 19,6 g 3-(trans-4-Butylcyclohexyl)propanal und 7,5 g Acrylsäureäthylester in 50 ml Dioxan zugetropft. Das Reaktionsgemisch wird noch 15 Stunden bei 80°C gerührt, dann auf Raumtemperatur abgekühlt und am Rotationsverdampfer vom Lösungsmittel befreit. Der Rückstand wird in 200 ml tert.-Butylmethyläther aufgenommen und nacheinander mit verdünnter Schwefelsäure, verdünnter Natriumhydrogencarbonat-Lösung und Wasser gewaschen. Die wässrigen Phasen werden

mit 100 ml tert.-Butylmethyläther nachextrahiert. Die vereinigten organischen Phasen werden über Natriumsulfat getrocknet und am Rotationsverdampfer vom Lösungsmittel befreit. Das erhaltene Rohprodukt von 6-(trans-4-Butylcyclohexyl)-4-oxo-capronsäureäthylester wird aus Essigsäureäthylester umkristallisiert.

d) 29,65 g 6-(trans-4-Butylcyclohexyl)-4-oxo-capronsäureäthylester werden in 300 ml Methanol gelöst, innert 10 Minuten tropfenweise mit 5,5 g Hydrazinhydrat versetzt und dann 2 Stunden am Rückfluss gekocht. Anschliessend wird das Reaktionsgemisch auf Raumtemperatur abgekühlt und am Rotationsverdampfer vom Lösungsmittel befreit. Der Rückstand von 6-[2-(trans-4-Butylcyclohexyl)äthyl]-4,5-dihydro-3(2H)-pyridazinon wird zweimal mit je 150 ml Toluol zur Trockene eingedampft und anschliessend unter Vakuum bei 40°C getrocknet.

e) 26,4 g 6-[2-(trans-4-Butylcyclohexyl)äthyl]-4,5-dihydro-3(2H)-pyridazinon werden in 75 ml Eisessig gelöst, auf 40°C erwärmt und dann ohne zusätzliches Erwärmen innert 30 Minuten tropfenweise mit 19,2 g Brom versetzt (exotherm). Anschliessend wird das Reaktionsgemisch noch 2 Stunden bei 60°C gerührt, auf Raumtemperatur gekühlt, mit 100 ml Essigsäureäthylester verdünnt und genutscht. Das Nutschgut wird mit Essigsäureäthylester ausgewaschen, in 200 ml Wasser aufgeschlämmt, bei 50°C mit Natriumhydrogencarbonat neutralisiert, auf 0°C gekühlt, genutscht und der Rückstand mit kaltem Wasser gewaschen. Die noch feuchten, gut abgepressten Kristalle von 6-[2-(trans-4-Butylcyclohexyl)äthyl]-3(2H)-pyridazinon werden schliesslich aus Aethanol umkristallisiert.

f) 21,0 g 6-[2-(trans-4-Butylcyclohexyl)äthyl]-3(2H)-pyridazinon werden mit 20 ml Phosphoroxychlorid versetzt und langsam bis zum Rückfluss erwärmt. Nach 20 Minuten Kochen unter Rückfluss wird das überschüssige Phosphoroxychlorid abdestilliert. Der Rückstand wird auf 0°C abgekühlt, vorsichtig mit 100 g zerstossenem Eis hydrolysiert, dann mit Natriumhydrogencarbonat neutralisiert und dreimal mit je 100 ml tert.-Butylmethyläther extrahiert. Die vereinigten organischen Phasen werden über Natriumsulfat getrocknet und am Rotationsverdampfer vom Lösungsmittel befreit. Das erhaltene Rohprodukt von 3-Chlor-6-[2-(trans-4-butylcyclohexyl)äthyl]pyridazin wird aus Aethanol umkristallisiert.

## Beispiel 2

a) Eine auf 0°C gekühlte Lösung von 90,9 g Tetrabrommethan in 100 ml Methylenchlorid wurde unter Argonbegasung innert 20 Minuten mit einer Lösung von 143,9 g Triphenylphosphin in 200 ml Methylenchlorid versetzt. Die klare, orange Reaktionslösung wurde noch 5 Minuten bei 0°C gerührt und dann innert 10 Minuten bei 0°C tropfenweise mit einer Lösung von 25 g trans-4-Pentylcyclohexancarboxaldehyd (Reinheit ca. 82%) in 80 ml Methylenchlorid versetzt. Nach 30 Minuten Rühren bei 0°C wurde das Reaktionsgemisch auf 2,5 l Hexan gegossen und der dabei ausgefallene Niederschlag abfiltriert. Das Filtrat wurde eingeengt, mit 1 l Hexan digeriert, filtriert und erneut eingeengt. Niederdruckchromatographie (0,5 bar) des Rückstandes (52,7 g) an Kieselgel mit Hexan ergab 35,9 g (77,4%) trans-1-(2,2-Dibromvinyl)-4-pentylcyclohexan als farbloses Oel; Rf-Wert (Hexan) 0,58.

b) Unter Argonbegasung wurde eine Lösung von 35,9 g trans-1-(2,2-Dibromvinyl)-4-pentylcyclohexan in 300 ml absolutem Tetrahydrofuran bei -70°C vorgelegt und innert 20 Minuten mit 222 ml einer 1,2M Lösung von Butyllithium in Hexan versetzt. Das Reaktionsgemisch wurde anschliessend noch 2 Stunden bei -70°C gerührt, dann auf 1,2 l Wasser gegossen und dreimal mit je 400 ml Hexan extrahiert. Die organischen Phasen wurden noch zweimal mit je 400 ml Wasser gewaschen, über Magnesiumsulfat getrocknet und eingeengt. Kristallisation des Rückstandes (19,4 g) aus 250 ml

Methanol bei -78°C ergab 10,0 g trans-1-Aethinyl-4-pentylcyclohexan, welches bei Raumtemperatur als farblose Flüssigkeit vorlag. Kristallisation der auf ca. 100 ml eingeengten Mutterlauge bei -78°C ergab weitere 3,9 g trans-1-Aethinyl-4-pentylcyclohexan. Die vereinigten Produktmengen wurden nochmals aus 300 ml Methanol bei -78°C kristallisiert und anschliessend im Kugelrohr flachdestilliert (110°C/0,3 Torr), wobei 10,43 g (55%) trans-1-Aethinyl-4-pentylcyclohexan (Reinheit 99,4%) als farblose Flüssigkeit erhalten wurden; Rf-Wert (Hexan) 0,41.

Beispiel 3

In einem Sulfierkolben wurden unter Stickstoff 8,0 g 3-Brom-6-propylpyridazin in 50 ml Triäthylamin gelöst. Die Lösung wurde auf 0°C gekühlt, mit 562 mg Bis-(triphenylphosphin)-palladium-(II)-dichlorid und 76 mg Kupfer-(I)-jodid versetzt und dann innert 20 Minuten tropfenweise mit 7,5 g trans-1-Aethinyl-4-pentylcyclohexan (hergestellt nach Beispiel 2) versetzt. Anschliessend wurde der Ansatz unter Kühlung mit einem Eisbad über Nacht gerührt und dann noch 3 Stunden auf 50°C erwärmt. Das Reaktionsgemisch wurde am Rotationsverdampfer eingeengt. Der Rückstand wurde in 100 ml Hexan aufgenommen und mit 100 ml Wasser gewaschen, wobei der pH-Wert der wässrigen Phase mit Ammoniumchlorid neutral gestellt wurde. Die wässrige Phase wurde noch zweimal mit je 50 ml Hexan nachextrahiert. Die vereinigten organischen Phasen wurden nochmals mit 100 ml Wasser gewaschen und die wässrige Phase mit 50 ml Hexan nachextrahiert. Die organischen Phasen wurden über Natriumsulfat getrocknet und am Rotationsverdampfer vom Lösungsmittel befreit. Chromatographie des erhaltenen, braunen, kristallisierten Oels an Kieselgel mit Hexan/Essigester (Vol. 2:1) ergab eine Fraktion des gewünschten Produkts 3-[2-(trans-4-Pentylcyclohexyl)äthinyl]-6-propylpyridazin (5,2 g) sowie eine Mischfraktion (4,8 g) bestehend aus 3-Brom-6-propylpyridazin und Produkt. Die reine Fraktion wurde aus 15 ml Aethanol bei -20°C umkristallisiert. Hierbei wurden 4,0 g weisse

Kristalle erhalten, welche nochmals aus 25 ml Pentan bei -20°C umkristallisiert wurden. Anschliessend wurden die Kristalle 2 Stunden in einem Vakuumtrockenschrank bei 40°C und dann über Nacht bei Raumtemperatur mit einem schwachen Stickstoffatom im Feinvakuum getrocknet, wobei 3,4 g 3-[2-(trans-4-Pentylcyclohexyl)äthinyl]-6-propylpyridazin als weisse Kristalle mit Smp. (C-I) 118-118,5°C erhalten wurden.

Das als Ausgangsmaterial verwendete 3-Brom-6-propylpyridazin wurde wie folgt hergestellt:

a) Ein Gemisch von 86 g Maleinsäurediäthylester, 144 g Butyraldehyd und 4 g Dibenzoylperoxid wurde 20 Stunden am Rückfluss gekocht. Nach beendeter Reaktion wurde zunächst der überschüssige Butyraldehyd über eine Vigreux-Kolonne abdestilliert und dann das gelb gefärbte Rohprodukt in 250 ml Hexan aufgenommen und mit 200 ml 5%iger (Gew./Vol.) Natriumhydrogencarbonat-Lösung und 200 ml Wasser gewaschen. Die wässrigen Phasen wurden mit wenig Hexan nachextrahiert. Die vereinigten organischen Phasen wurden über Natriumsulfat getrocknet und am Rotationsverdampfer vom Lösungsmittel befreit. Destillation des erhaltenen Rohproduktes im Feinvakuum über eine Vigreux-Kolonne ergab 104,5 g (85,6%) 2-Butyrylsuccinsäurediäthylester als schwach gelb gefärbte Flüssigkeit; Sdp. 84-90°C/0,08 Torr.

b) In einem Rundkolben mit Destillationsaufsatz wurden 92,8 g 2-Butyrylsuccinsäurediäthylester und 23,5 g Borsäure auf 150°C erwärmt, wobei Aethanol abdestillierte. Anschliessend wurde das Reaktionsgemisch auf 175°C erhitzt und 4 Stunden bei dieser Temperatur gerührt (Entweichen von Kohlendioxid). Nach beendeter Reaktion wurde das Reaktionsgemisch auf Raumtemperatur gekühlt, in 500 ml Hexan aufgenommen und mit 150 ml 5%iger (Gew./Vol.) Natriumhydrogencarbonat-Lösung und 100 ml Wasser gewaschen. Die wässrigen Phasen wurden mit 50 ml Hexan nachextrahiert. Die vereinigten organischen Phasen wurden über Natriumsulfat getrocknet und am Rotationsverdampfer vom Lösungsmittel befreit.

Destillation des erhaltenen Rohproduktes im Feinvakuum über eine Vigreux-Kolonne ergab im Hauptlauf 58,6 g (89,6%) 4-Oxoheptansäureäthylester als farblose Flüssigkeit; Sdp. 50-56°C/0,2 Torr.

c) 56,8 g 4-Oxoheptansäureäthylester wurden in 150 ml Methanol gelöst und innert 20 Minuten tropfenweise mit 18,0 g Hydrazinhydrat versetzt, wobei die Temperatur auf 38°C anstieg. Das Reaktionsgemisch wurde noch 1,5 Stunden am Rückfluss gekocht und dann am Rotationsverdampfer vom Lösungsmittel befreit. Die zurückbleibende Flüssigkeit wurde in 300 ml Chloroform aufgenommen und mit 150 ml Wasser gewaschen. Die Wasserphase wurde dreimal mit je 100 ml Chloroform nachextrahiert. Die vereinigten organischen Phasen wurden über Natriumsulfat getrocknet und am Rotationsverdampfer vom Lösungsmittel befreit. Das erhaltene helle Oel wurde in 200 ml Toluol aufgenommen und am Rotationsverdampfer bis zur Gewichtskonstanz eingedampft. Hierbei wurden 48,6 g 6-Propyl-4,5-dihydro-3(2H)-pyridazinon als schwach gefärbtes Oel erhalten, welches vermutlich noch Hydratwasser enthielt; Sdp. 110-120°C/0,03 Torr.

d) 48,6 g 6-Propyl-4,5-dihydro-3(2H)-pyridazinon wurden in 90 ml Eisessig gelöst, auf 70°C erwärmt und innert 40 Minuten tropfenweise mit 63,3 g Brom versetzt, wobei ein Temperaturanstieg auf 107°C und eine starke Gasentwicklung auftrat. Nach beendeter Bromzugabe wurde das Reaktionsgemisch noch 1 Stunde bei 80°C gerührt, dann auf Raumtemperatur abgekühlt und am Rotationsverdampfer der Eisessig abgezogen. Der dunkle, ölige Rückstand wurde in 600 ml Wasser aufgenommen und zuerst mit festem Natriumhydroxid und dann mit Natriumhydrogencarbonat neutralisiert. Am Boden des Gefässes schied sich ein fast schwarzes Oel ab. Die überstehende trübe Lösung wurde abgetrennt, 15 Minuten bei 40°C mit Aktivkohle verrührt, genutscht und mit 100 ml warmem Wasser nachgewaschen. Das klare Filtrat

wurde auf Raumtemperatur gekühlt und einmal mit 500 ml Chloroform und zweimal mit je 250 ml Chloroform extrahiert. Die vereinigten organischen Phasen wurden über Natriumsulfat getrocknet und am Rotationsverdampfer vom Lösungsmittel befreit. Die erhaltenen, braun gefärbten Kristalle wurden dreimal mit je 300 ml Toluol bis zur Trockene eingedampft und dann aus 140 ml tert.-Butylmethyläther bei -20°C umkristallisiert. Hierbei wurden 28,0 g fast weisse Kristalle erhalten. Durch Einengen der Mutterlauge und Umkristallisation aus 36 ml tert.-Butylmethyläther bei -20°C wurden weitere 8,2 g hellbeige Kristalle erhalten. Ausbeute: 36,2 g (78,9%) 6-Propyl-3(2H)-pyridazinon mit Smp. 51-60°C.

e) In einem Sulfierkolben wurden unter Stickstoff 11,75 g 6-Propyl-3(2H)-pyridazinon in 70 ml absolutem Toluol warm gelöst, auf 80°C erwärmt und unter gutem Rühren innert 30 Minuten tropfenweise mit einer Lösung von 24,4 g Phosphoroxybromid in 24,4 g absolutem Toluol versetzt. Anschliessend wurde der Ansatz noch 3 Stunden unter Rückfluss gerührt. Nach beendeter Reaktion wurde das Reaktionsgemisch auf 200 ml Wasser gegossen und mit festem Natriumhydrogencarbonat neutralisiert. Die Phasen wurden getrennt und die wässrige Phase zweimal mit je 100 ml tert.-Butylmethyläther nachextrahiert. Die vereinigten organischen Phasen wurden mit 100 ml Wasser gewaschen und über Natriumsulfat getrocknet. Nach Abdampfen des Lösungsmittels wurde der dunkelbraune, kristalline Rückstand (14,1 g) bei 40-60°C im Feinvakuum sublimiert, wobei 8,2 g (48%) 3-Brom-6-propylpyridazin als weisse Kristalle mit Smp. 35-36°C erhalten wurden.

In analoger Weise wurde folgende Verbindung hergestellt:

3-[2-(trans-4-Pentylcyclohexyl)äthinyl]-6-methylpyridazin; Smp. (C-I) 92,5-93°C,

2-Hexanoylsuccinsäurediäthylester; Sdp. 108-112°C/ 0,1 Torr,

4-Oxononansäureäthylester, Sdp. 75-80°C/0,2 Torr,
6-Pentyl-4,5-dihydro-3(2H)-pyridazinon; Smp. 34°C,
6-Pentyl-3(2H)-pyridazinon, Smp. 40°C.

## Beispiel 4

In einem Sulfierkolben wurden unter Stickstoff 8,0 g 3-Brom-6-propylpyridazin (hergestellt nach Beispiel 3) in 50 ml Triäthylamin gelöst. Die Lösung wurde auf 0°C gekühlt, mit 562 mg Bis-(triphenylphosphin)-palladium-(II)-dichlorid und 76 mg Kupfer-(I)-jodid versetzt und dann innert 20 Minuten tropfenweise mit 7,5 g trans-1-Aethinyl-4-pentylcyclohexan versetzt. Anschliessend wurde der Ansatz noch einige Stunden bei 0°C, dann über Nacht bei Raumtemperatur und schliesslich noch 3 Stunden bei 50°C gerührt. Zur Aufarbeitung wurde der dunkel gefärbte Ansatz mit 100 ml tert.Butylmethyläther verdünnt und durch ein Papierfilter genutscht (Nachwaschen mit tert.Butylmethyläther). Das Filtrat wurde am Rotationsverdampfer eingeengt. Der dunkel gefärbte, kristalline Rückstand wurde in 150 ml tert.Butylmethyläther aufgenommen und nacheinander mit 100 ml 2%-iger (Gew./Vol.) Ammoniumchloridlösung und mit 100 ml Wasser gewaschen. Die wässrigen Phasen wurden jeweils mit wenig tert.Butylmethyläther nachextrahiert. Die vereinigten organischen Phasen wurden über Natriumsulfat getrocknet und am Rotationsverdampfer eingeengt. Zur Reinigung wurden die erhaltenen, braun gefärbten Kristalle an Kieselgel mit Hexan/Essigester (Vol. 2:1) chromatographiert, wobei eine Fraktion des gewünschten Produkts 3-[2-(trans-4-Pentylcyclohexyl)äthinyl]-6-propylpyridazin (5,2 g) und eine Mischfraktion bestehend aus 3-Brom-6-propylpyridazin und Produkt (4,8 g) erhalten wurden. Die reine Fraktion wurde einmal aus Aethanol bei -20°C und einmal aus Hexan bei -20°C umkristallisiert und schliesslich im Vakuumtrockenschrank bei 40°C gut getrocknet. Hierbei wurden 3,4 g (28,5%) 3-[2-(trans-4-Pentylcyclohexyl)äthinyl]-6-propylpyridazin in Form weisser Kristalle mit Smp. (C-I) 118,5°C, Sdp. 210-220°c/ 0,08 Torr erhalten.

Das als Ausgangsmaterial verwendete trans-1-Aethinyl-4-pentylcyclohexan wurde wie folgt hergestellt:

a) Eine auf -20°C gekühlte Lösung von 331,6 g Tetrabrommethan in 1 l trockenem Methylenchlorid wurde unter Stickstoffbegasung innert 30 Minuten tropfenweise mit einer Lösung von 524,6 g Triphenylphosphin in 1 l Methylenchlorid versetzt. Anschliessend wurde das Gemisch noch 30 Minuten bei -5°C gerührt, dann bei dieser Temperatur innert 30 Minuten tropfenweise mit einer Lösung von 91,2 g trans-4-Pentylcyclohexancarboxaldehyd in 500 ml Methylenchlorid versetzt und noch 2 Stunden bei -5°C gerührt. Der Ansatz wurde auf 6 l Hexan gegossen, das Gemisch 1 Stunde bei -20°C gerührt und dann der Niederschlag genutscht und mit ca. 1 l kaltem (-20°C) Hexan gut ausgewaschen. Das Filtrat wurde am Rotationsverdampfer auf ein Volumen von ca. 1 l eingeengt, mit 2 l Hexan verdünnt, wiederum auf ein Volumen von ca. 1 l eingeengt und noch 1 Stunde bei -20°C verrührt. Der Niederschlag wurde genutscht und mit ca. 500 ml kaltem (-20°C) Hexan gut ausgewaschen. Das Filtrat wurde am Rotationsverdampfer vom Lösungsmittel befreit und das erhaltene gelbe Oel an Kieselgel mit ca. 3 l Hexan chromatographisch filtriert. Das Eluat wurde am Rotationsverdampfer bis zur Gewichtskonstanz eingedampft, wobei 122,8 g (72,6%) trans-1-(2,2-Dibromvinyl)-4-pentylcyclohexan als farbloses Oel erhalten wurden; Rf-Wert (Hexan) 0,55.

b) Unter Stickstoffbegasung wurde eine Lösung von 84,5 g trans-1-(2,2-Dibromvinyl)-4-pentylcyclohexan in 800 ml absolutem Tetrahydrofuran auf -75°C gekühlt und innert 30 Minuten tropfenweise mit 400 ml einer ca. 1,6M Lösung von Butyllithium in Hexan versetzt. Das Reaktionsgemisch wurde anschliessend noch 2 Stunden bei -70°C gerührt, dann auf 5,5 l 2%-ige (Gew./Vol.) Natriumhydrogencarbonat-Lösung gegossen und mit 1 l Hexan extrahiert. Die wässrige Phase wurde mit 700 ml Hexan nachextrahiert. Die vereinigten organischen Phasen wurden über Natriumsulfat getrocknet und am Rotationsverdampfer vom Lösungsmittel befreit.

Destillation des erhaltenen hellgelben Oeles im Feinvakuum ergab im Hauptlauf 40,6 g (91,1%) trans-1-Aethinyl-4-pentylcyclohexan als farblose Flüssigkeit, welche noch cis-Isomer enthielt; Sdp. 52-54°C/0,02 Torr. Durch Umkristallisation aus Aethanol bei -25°C und Trocknen bei 30°C im Vakuumtrockenschrank bis zur Gewichtskonstanz konnten 20,4 g cis-freies Produkt erhalten werden. Aus der Mutterlauge konnten nach derselben Methode weitere 6,5 g fast cis-freies Produkt isoliert werden.

Beispiel 5

In einem Sulfierkolben wurden unter Stickstoff 7,8 g 3-Chlor-6-propylpyridazin in 30 ml Triäthylamin gelöst. Die Lösung wurde auf 0°C gekühlt, mit 700 mg Bis-(triphenylphosphin)-palladium-(II)-dichlorid und 95 mg Kupfer-(I)-jodid versetzt und dann innert 30 Minuten tropfenweise mit einer Lösung von 8,4 g trans-1-Aethinyl-4-butylcyclohexan in 10 ml Triäthylamin versetzt. Anschliessend wurde der Ansatz noch 3 Stunden bei Raumtemperatur und dann über Nacht bei 50°C gerührt. Zur Aufarbeitung wurde der dunkel gefärbte Ansatz mit 150 ml tert.Butylmethyläther verdünnt und durch ein Papierfilter genutscht (Nachwaschen mit tert.Butylmethyläther). Das Filtrat wurde am Rotationsverdampfer eingeengt. Der dunkel gefärbte, kristalline Rückstand wurde in 150 ml tert.Butylmethyläther aufgenommen und nacheinander mit 100 ml 2%-iger (Gew./Vol.) Ammoniumchloridlösung und mit 100 ml Wasser gewaschen. Die wässrigen Phasen wurden jeweils mit wenig tert.Butylmethyläther nachextrahiert. Die vereinigten organischen Phasen wurden über Natriumsulfat getrocknet und am Rotationsverdampfer eingeengt. Destillation des erhaltenen Rohproduktes im Feinvakuum ergab im Hauptlauf (Sdp. 190°C/0,05 Torr) 10,2 g Produkt in Form gelber Kristalle. Umkristallisation dieses Produktes aus Aceton bei -20°C und Aufarbeitung der Mutterlauge ergab insgesamt 7,6 g hellbeige Kristalle. Diese wurden nochmals aus 22 ml Aceton

bei -20°C umkristallisiert, wobei 5,5 g (38,7%) 3-[2-(trans-4-Butylcyclohexyl)äthinyl]-6-propylpyridazin in Form weisser Kristalle mit Smp. (C-I) 117°C erhalten wurden.

Das als Ausgangsmaterial verwendete 3-Chlor-6-propylpyridazin wurde wie folgt hergestellt:

a) In einem Sulfierkolben wurden unter Stickstoff 46,0 g Phosphoroxychlorid auf 60°C erwärmt und innert 1 Stunde mit 27,6 g 6-Propyl-3(2H)-pyridazinon (hergestellt nach Beispiel 3) in kleinen Portionen versetzt. Anschliessend wurde der Ansatz langsam bis auf 100°C erwärmt und bei dieser Temperatur gerührt, bis die Reaktion gemäss Dünnschichtchromatogramm beendet war (ca. 2 Stunden). Nach dem Abkühlen auf Raumtemperatur wurde die dunkelbraune Lösung auf 1 l Wasser gegossen und mit festem Natriumhydroxid und Natriumhydrogencarbonat neutralisiert. Anschliessend wurde die wässrige Phase einmal mit 500 ml und dann zweimal mit je 250 ml tert.Butylmethyläther extrahiert. Die organischen Phasen wurden mit 500 ml Wasser gewaschen und die wässrige Phase zweimal mit je 100 ml tert.Butylmethyläther nachextrahiert. Die vereinigten organischen Phasen wurden über Natriumsulfat getrocknet und am Rotationsverdampfer vom Lösungsmittel befreit. Destillation des erhaltenen dunkelbraunen Oeles im Feinvakuum ergab im Hauptlauf 22,9 g (73,1%) 3-Chlor-6-propylpyridazin als farblose Flüssigkeit; Sdp. 72-74°C/0,4 Torr.

Das als Ausgangsmaterial verwendete trans-1-Aethinyl-4-butylcyclohexan wurde wie folgt hergestellt:

b) Eine auf -20°C gekühlte Lösung von 398 g Tetrabrommethan in 1 l Methylenchlorid wurde unter Stickstoffbegasung innert 30 Minuten tropfenweise mit einer Lösung von 629,6 g Triphenylphosphin in 1 l Methylenchlorid versetzt. Anschliessend wurde das Gemisch noch 30 Minuten bei -10°C gerührt, dann innert 30 Minuten tropfenweise mit einer Lösung von 101 g trans-4-Butylcyclohexancarboxaldehyd in

500 ml Methylenchlorid versetzt und noch 2 Stunden bei -5°C gerührt. Der Ansatz wurde auf 4 l Hexan gegossen, das Gemisch 1 Stunde bei -20°C gerührt und dann der Niederschlag genutscht und mit 1 l kaltem (-20°C) Hexan gut ausgewaschen. Das Filtrat wurde am Rotationsverdampfer auf ein Volumen von ca. 1 l eingeengt, mit 2 l Hexan verdünnt, wiederum auf ein Volumen von ca. 1 l eingeengt und noch 30 Minuten bei -20°C verrührt. Der Niederschlag wurde genutscht und mit 700 ml kaltem (-20°C) Hexan gut ausgewaschen. Das Filtrat wurde am Rotationsverdampfer vom Lösungsmittel befreit und das erhaltene gelbe Oel (195 g) an Kieselgel mit ca. 3 l Hexan chromatographisch filtriert. Das Eluat wurde am Rotationsverdampfer bis zur Gewichtskonstanz eingedampft, wobei 171,3 g (88,1%) trans-1-(2,2-Dibromvinyl)-4-butylcyclohexan als farblose Flüssigkeit erhalten wurden; Rf-Wert (Hexan) 0,55.

c) Unter Stickstoffbegasung wurde eine Lösung von 162,1 g trans-1-(2,2-Dibromvinyl)-4-butylcyclohexan in 1,3 l absolutem Tetrahydrofuran auf -75°C gekühlt und innert 1 Stunde tropfenweise mit 690 ml einer 1,6M Lösung von Butyllithium in Hexan versetzt. Das Reaktionsgemisch wurde anschliessend noch 2 Stunden bei -70°C gerührt, dann auf 1,5 l Wasser gegossen, mit konzentrierter Salzsäure neutralisiert und zweimal mit je 1 l Hexan extrahiert. Die vereinigten organischen Phasen wurden mit 500 ml Wasser gewaschen, über Natriumsulfat getrocknet und am Rotationsverdampfer bis zur Trockene eingeengt. Destillation des erhaltenen, hellgelben Rohproduktes im Feinvakuum ergab im Hauptlauf 75,9 g (92,4%) trans-1-Aethinyl-4-butylcyclohexan als farblose Flüssigkeit; Sdp. 41-43°C/0,4 Torr.

In analoger Weise wurde folgende Verbindung hergestellt:

3-Chlor-6-pentylpyridazin; Sdp. 82°C/0,04 Torr.

Beispiel 6

In einem Sulfierkolben wurden unter Stickstoff 11,1 g 3-Chlor-6-pentylpyridazin in 40 ml Triäthylamin gelöst. Die Lösung wurde auf 0°C gekühlt, mit 840 mg Bis-(triphenylphosphin)-palladium-(II)-dichlorid und 114 mg Kupfer-(I)-jodid versetzt und dann innert 30 Minuten tropfenweise mit einer Lösung von 9,1 g trans-1-Aethinyl-4-pentylcyclohexan in 15 ml Triäthylamin versetzt. Anschliessend wurde der Ansatz über Nacht bei Raumtemperatur gerührt und dann nach Zugabe von 2,35 g trans-1-Aethinyl-4-pentylpyridazin nochmals über Nacht bei 50°C gerührt. Zur Aufarbeitung wurde der Ansatz in 180 ml tert.Butylmethyläther aufgenommen und durch ein Papierfilter genutscht (Nachwaschen mit tert.Butylmethyläther). Das Filtrat wurde am Rotationsverdampfer eingeengt. Der dunkel gefärbte, ölige Rückstand wurde in 200 ml tert.Butylmethyläther aufgenommen und zweimal mit je 100 ml Wasser gewaschen. Die wässrigen Phasen wurden mit 50 ml tert.Butylmethyläther nachextrahiert. Die vereinigten organischen Phasen wurden über Natriumsulfat getrocknet und am Rotationsverdampfer bis zur Trockene eingedampft. Umkristallisation des erhaltenen Rohproduktes aus 70 ml Hexan bei -20°C und Aufarbeitung der Mutterlauge ergab insgesamt 14,0 g beige Kristalle. Diese wurden nochmals aus 42 ml Aethanol bei -20°C umkristallisiert, wobei 10,7 g (45,6%) 3-[2-(trans-4-Pentylcyclohexyl)äthinyl]-6-pentylpyridazin als hellbeige Kristalle mit Smp. (C-I) 108-109°C erhalten wurden.

Beispiel 7

In einem Rundkolben wurden 3,6 g 3-[2-(trans-4-Pentylcyclohexyl)äthinyl]-6-propylpyridazin in 40 ml Methanol gelöst, mit 0,2 g Palladium/Kohle (5%) versetzt und bei Raumtemperatur unter Schütteln hydriert (Wasserstoffaufnahme: 660 ml in ca. 4 Stunden). Nach beendeter Reaktion wurde das Gemisch genutscht, der Filterrückstand mit Methanol gut ausgewaschen und das Filtrat am Rotationsverdampfer vom Lösungsmittel befreit. Kugelrohrdestillation des erhaltenen Rohproduktes ergab bei 175-180°C/0,05 Torr 3,4 g Produkt in Form gelblicher Kristalle. Diese wurden aus 15 ml Aceton bei -20°C umkristallisiert, wobei 2,5 g weisse Kristalle erhalten wurden. Aus der Mutterlauge wurden nach Einengen und Umkristallisation aus 4 ml Aceton weitere 0,4 g weisse Kristalle erhalten. Beide Kristallisate (2,9 g) wurden nochmals aus 12,5 ml Aceton bei -20°C umkristallisiert. Die erhaltenen weissen Kristalle (2,6 g) wurden schliesslich in 50 ml Hexan gelöst, filtriert, eingeengt, aus 26 ml Hexan bei -20°C umkristallisiert und im Hochvakuum in einem schwachen Stickstoffstrom bei Raumtemperatur über Nacht getrocknet. Ausbeute: 2,3 g (63,9%) 3-[2-(trans-4-Pentylcyclohexyl)äthyl]-6-propylpyridazin in Form weisser Kristalle mit Smp. 64,2°C.

In analoger Weise wurden folgende Verbindungen hergestellt:

3-[2-(trans-4-Butylcyclohexyl)äthyl]-6-propylpyridazin; Smp. 67,5°C,

3-[2-(trans-4-Pentylcyclohexyl)äthyl]-6-pentylpyridazin; Smp. 80,9°C.

## Beispiel 8

a) 116,1 g Laevulinsäure wurden in 400 ml Methanol gelöst und innert 15 Minuten tropfenweise mit 51,0 g Hydrazinhydrat versetzt. In exothermer Reaktion stieg die Temperatur auf 53°C an. Zur Vervollständigung der Reaktion wurde das Gemisch noch 2 Stunden bei 60°C nachgerührt, dann auf Raumtemperatur gekühlt und am Rotationsverdampfer vom Lösungsmittel befreit. Der ölige Rückstand wurde einmal mit 200 ml Toluol und einmal mit 200 ml Isopropanol bis zur Trockene eingedampft. Nach weiterer Trocknung im Vakuumtrockenschrank bei 50°C bis zur Gewichtskonstanz wurden 109,9 g (98%) 6-Methyl-4,5-dihydro-3(2H)-pyridazinon als schwach gelb gefärbte Kristalle mit Smp. 102-103°C erhalten.

b) Eine Lösung von 112,1 g 6-Methyl-4,5-dihydro-3(2H)-pyridazinon in 300 ml Eisessig wurde auf 40°C erwärmt und dann ohne weiteres Erwärmen innert 1,5 Stunden tropfenweise mit 199,8 g Brom versetzt. In exothermer Reaktion stieg die Temperatur bis auf 80°C an. Nach Zugabe von etwas mehr als der Hälfte des Broms begann ein weisser Niederschlag auszukristallisieren. Der Ansatz wurde noch 3 Stunden bei 80°C gerührt, dann auf Raumtemperatur gekühlt, mit 300 ml Essigsäureäthylester verdünnt und genutscht (Nachwaschen mit 250 ml Essigsäureäthylester). Die gelb gefärbten Kristalle wurden in 250 ml Wasser aufgeschlämmt und die Suspension mit 100 ml 28%-iger (Gew./Vol.) Natronlauge und festem Natriumhydrogencarbonat neutralisiert, mit 50 ml Wasser verdünnt und auf dem Dampfbad bis zur vollständigen Lösung der Kristalle erwärmt. Durch langsames Abkühlen bis auf 0°C kristallisierte das Produkt wieder aus. Dieses wurde genutscht, mit 200 ml kaltem Wasser gut ausgewaschen und in 300 ml Methanol heiss gelöst. Die Lösung wurde filtriert und das Filtrat langsam bis auf -20°C gekühlt. Das auskristallisierte Produkt wurde abgenutscht und mit kaltem Methanol gewaschen. Einengen und Umkristallisation der Mutterlauge aus 100 ml Methanol bei -20°C ergab weiteres Produkt. Beide Kristallisate wurden bei 60°C im Vakuumtrockenschrank bis zur Gewichtskonstanz getrocknet, wobei 87,7 g (79,6%) 6-Methyl-3(2H)-pyridazinon als weisse Kristalle mit Smp. 141-142°C erhalten wurden.

c) 34,5 g Phosphoroxychlorid wurden unter Stickstoff auf 70°C erwärmt und innert 1 Stunde mit 16,5 g 6-Methyl-3(2H)-pyridazinon in kleinen Portionen versetzt. Nach beendeter Zugabe wurde die Temperatur auf 90°C erhöht und der Ansatz noch ca. 2 Stunden gerührt. Zur Aufarbeitung wurde die dunkelbraune Lösung auf 300 ml Wasser gegossen, gut verrührt und mit festem Natriumhydroxid und Natriumhydrogencarbonat neutralisiert. Anschliessend wurde die wässrige Phase mit 500 ml Hexan über Nacht kontinuierlich extrahiert. Die organische Phase wurde abgetrennt und vom Lösungsmittel befreit. Die erhaltenen braun gefärbten Kristalle wurden aus einem 50-60°C warmen Bad sublimiert. Hierbei wurden 12,8 g (66,4%) 3-Chlor-6-methylpyridazin als weisse Kristalle mit Smp. 59-60°C erhalten.

## Beispiel 9

In einem Sulfierkolben wurde unter Stickstoff eine Lösung von 18,6 g Phosphoroxybromid in 18,6 g absolutem Toluol mit 100 ml absolutem Toluol verdünnt, auf 65°C erwärmt und innert 1,5 Stunden tropfenweise mit einer Lösung von 21,6 g 6-Pentyl-3(2H)-pyridazinon (hergestellt nach Beispiel 3) in 100 ml absolutem Toluol versetzt. Zeitweise bildete sich eine grau gefärbte, schwer rührbare Suspension. Zur Vervollständigung der Reaktion wurde der Ansatz noch über Nacht (18 Stunden) bei 65°C gerührt, dann auf Raumtemperatur gekühlt, auf 250 ml Wasser gegossen und gut verrührt. Die wässrige Phase wurde mit 28%-iger (Gew./Vol.) Natronlauge und mit Natriumhydrogencarbonat neutralisiert. Die organische Phase wurde abgetrennt, und die wässrige Phase wurde mit 100 ml tert.Butylmethyläther nachextrahiert. Die vereinigten organischen Phasen wurden mit 100 ml Wasser gewaschen, über Natriumsulfat getrocknet und am Rotationsverdampfer eingedampft. Anschliessend wurde das braun gefärbte Rohprodukt im Feinvakuum flachdestilliert, wobei 21,4 g (72%) 3-Brom-6-pentylpyridazin als braunes Oel erhalten wurden (Sdp. 99-108°C/0,04-0,06 Torr), welches beim Abkühlen kristallisierte. Umkristallisation einer Probe aus Ameisensäuremethylester bei -20°C ergab weisse Kristalle mit Smp. 31-32°C.

## Patentansprüche

1. Verbindungen der allgemeinen Formel

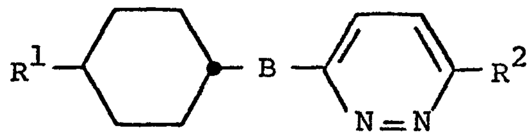

I

worin B die Gruppe $-C{\equiv}C-$ oder $-CH_2CH_2-$ darstellt, $R^1$ geradkettiges $C_1$-$C_{12}$-Alkyl bezeichnet und $R^2$ geradkettiges $C_1$-$C_{12}$-Alkyl oder geradkettiges $C_1$-$C_{12}$-Alkoxy bedeutet.

2. Verbindungen nach Anspruch 1, dadurch gekennzeichnet, dass B die Gruppe $-CH_2CH_2-$ darstellt.

3. Verbindungen nach Anspruch 1, dadurch gekennzeichnet, dass B die Gruppe $-C{\equiv}C-$ darstellt.

4. Verbindungen nach einem der Ansprüche 1 bis 3, dadurch gekennzeichnet, dass $R^2$ geradkettiges $C_1$-$C_{12}$-Alkyl bedeutet.

5. Verbindungen nach einem der Ansprüche 1 bis 4, dadurch gekennzeichnet, dass $R^1$ geradkettiges $C_3$-$C_7$-Alkyl bedeutet.

6. Flüssigkristallines Gemisch enthaltend ein flüssigkristallines Trägermaterial und mindestens eine Verbindung der in Anspruch 1 definierten Formel I.

7. Flüssigkristallines Gemisch nach Anspruch 6 bestehend aus einem flüssigkristallinen Trägermaterial mit einer dielektrischen Anisotropie von höchstens +1 und mindestens einer Verbindung der Formel I.

8. Flüssigkristallines Gemisch nach Anspruch 6, dadurch gekennzeichnet, dass das Gemisch aus 3 Komponenten A, B und C besteht, welche jeweils eine oder mehrere Verbindungen enthalten, und dass Komponente A eine Viskosität von höchstens 40 cP, einen Klärpunkt von mindestens 40°C und eine dielektrische Anisotropie zwischen -2 und +1 aufweist, Komponente B eine dielektrische Anisotropie unterhalb -2 besitzt und mindestens eine Verbindung der Formel I enthält, und Komponente C eine dielektrische Anisotropie oberhalb +10, einen Klärpunkt von mindestens 100°C und eine Cross-over-Frequenz im Gesamtgemisch von höchstens 15 kHz bei 20°C aufweist.

9. Verfahren zur Herstellung der Verbindungen der in Anspruch 1 definierten Formel I, dadurch gekennzeichnet, dass man

a) zur Herstellung der Verbindungen der Formel I, worin B die Gruppe -C≡C- darstellt, eine Verbindung der allgemeinen Formel

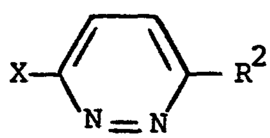

IV

worin X Chlor, Brom oder Jod bezeichnet und $R^2$ die in Anspruch 1 gegebene Bedeutung hat,

in Gegenwart von Kupfer-(I)-jodid, einer Triphenylphosphinpalladiumverbindung und eines Amins mit einer Verbindung der allgemeinen Formel

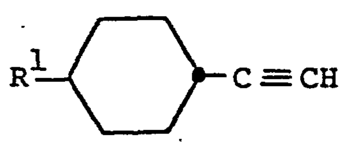

V

worin $R^1$ die in Anspruch 1 gegebene Bedeutung hat,

umsetzt, oder

b) zur Herstellung der Verbindungen der Formel I, worin B die Gruppe $-C\equiv C-$ und $R^2$ geradkettiges $C_1$-$C_{12}$-Alkoxy bedeutet, eine Verbindung der allgemeinen Formel

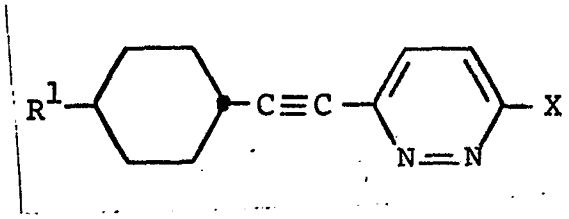 VI

worin X Chlor, Brom oder Jod bedeutet und $R^1$ die in Anspruch 1 gegebene Bedeutung hat,

mit Alkalimetallalkanolat umsetzt, oder

c) zur Herstellung der Verbindungen der Formel I, worin B die Gruppe $-CH_2CH_2-$ und $R^2$ geradkettiges $C_1$-$C_{12}$-Alkyl bedeuten, eine Verbindung der allgemeinen Formel

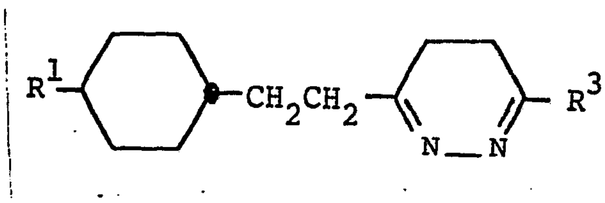 II

worin $R^3$ geradkettiges $C_1$-$C_{12}$-Alkyl bezeichnet und $R^1$ die in Anspruch 1 gegebene Bedeutung hat,

oder ein tautomeres Dihydropyridazin oxidiert, oder

d) zur Herstellung der Verbindungen der Formel I, worin B die Gruppe $-CH_2CH_2-$ und $R^2$ geradkettiges $C_1$-$C_{12}$-Alkoxy bedeuten, eine Verbindung der allgemeinen Formel

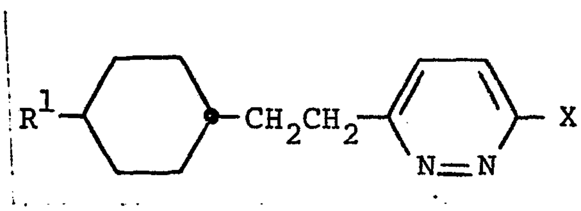 III

worin X Chlor, Brom oder Jod bezeichnet und $R^1$ die in Anspruch 1 gegebene Bedeutung hat,

mit Alkalimetallalkanolat umsetzt, oder

e) zur Herstellung der Verbindungen der Formel I, worin B die Gruppe $-CH_2CH_2-$ darstellt, eine Verbindung der Formel I, worin B die Gruppe -C≡C- darstellt, katalytisch hydriert.

10. Verwendung der Verbindungen der in Anspruch 1 definierten Formel I für elektro-optische Zwecke.

***